# EUROPEAN PATENT APPLICATION

(11) **EP 4 775 240 A1**
(43) Date of publication of application: **15.07.2026**
(21) Application number: 24862483.5
(22) Date of filing: 02.08.2024
(51) Int. Cl.: A61M 5/28, A61M 5/20, A61M 5/32

(54) **MEDICINE CARTRIDGE AND MEDICINE INJECTION DEVICE**

(30) Priority: 08.09.2023 JP 2023146124
(71) Applicant: PHC Holdings Corporation, Tokyo 100-8403 (JP)
(72) Inventor: FUJII, Yoshiyuki, Toon-shi, Ehime 791-0395 (JP); YASUI, Shinichi, Toon-shi, Ehime 791-0395 (JP); MURAKAMI, Kenji, Toon-shi, Ehime 791-0395 (JP)
(74) Representative: Eisenführ Speiser
(86) International application number: PCT/JP2024/027724
(87) International publication number: WO 2025/052829

(57) **Abstract**

This medicine cartridge is used upon being incorporated in a medicine injection device, and is provided with: a syringe that accommodates a medicine, a case that accommodates the syringe in a state in which the syringe can move in the front-rear direction, a cap that is attached to the front end part of the case, and a spring that is provided between the syringe and the case and constantly impels the syringe rearward.

## Description

### Technical Field

The present invention relates to a drug cartridge and a drug injection device.

### Background Art

Conventionally, a drug injection device used by a user (that is, a patient) to inject a drug by oneself is known (see PTL 1). Such a drug injection device is used with a disposable cartridge attached. The cartridge accommodates a syringe filled with a drug.

### Citation List

### Patent Literature

PTL 1
Japanese Unexamined Patent Application Publication (Translation of PCT Application) No. 2014-516634

### Summary of Invention

### Technical Problem

The drug cartridge and the drug injection device as described above are carried by the user and used at various places. Therefore, it is desirable that the drug cartridge and the drug injection device have excellent portability.

The present invention has been made in view of such a situation, and objectives thereof include providing a drug cartridge and a drug injection device having excellent portability.

### Solution to Problem

One aspect of a drug cartridge according to the present invention is a drug cartridge configured to be incorporated into and used with a drug injection device, the drug cartridge including:
a syringe configured to accommodate a drug;
a case configured to accommodate the syringe such that the syringe is movable in a front-rear direction;
a cap attached to a front end portion of the case; and
a spring disposed between the syringe and the case and configured to constantly bias the syringe rearward.

One aspect of a drug injection device according to the present invention is a drug injection device, including:
a casing including a housing configured to accommodate a drug cartridge configured to hold a syringe;
a motor accommodated in the casing; and
an actuator accommodated in the casing, wherein
the actuator performs, based on power of the motor,
a puncturing operation of moving the syringe in a front-rear direction,
an injection operation of moving a piston of the syringe forward, and
a locking operation of locking the drug cartridge.

### Advantageous Effects of Invention

According to the present invention, a drug cartridge and a drug injection device having excellent portability can be provided.

### Brief Description of Drawings

FIG. 1 is a perspective view of a drug cartridge and a drug injection device according to Embodiment 1;
FIG. 2 is a block diagram showing a configuration of the drug injection device;
FIG. 3 is an exploded perspective view of the drug cartridge;
FIG. 4 is a sectional view of the drug cartridge;
FIG. 5 is a sectional view for describing an assembly step of the drug cartridge;
FIG. 6 is a sectional view for describing an assembly step of the drug cartridge;
FIG. 7 is a sectional view of the drug cartridge;
FIG. 8 is a perspective view showing a configuration of a part of the drug injection device;
FIG. 9 is a perspective view showing a configuration of a part of the drug injection device;
FIG. 10A is a sectional view of a basic state of the drug injection device;
FIG. 10B is a sectional view of the drug injection device after a locking operation;
FIG. 10C is a sectional view of the drug injection device after a puncturing operation;
FIG. 10D is a sectional view of the drug injection device after an injection operation;
FIG. 10E is a sectional view of a state in which the needle withdrawal of the drug injection device has been completed (immediately after the needle withdrawal);
FIG. 10F is a sectional view of the drug injection device after the needle withdrawal operation (after all the needle withdrawal operations are completed);
FIG. 11A is an enlarged sectional view showing a state of a device-side engager, a locking engager, and a latch cover in a non-locked state of the drug injection device;
FIG. 11B is an enlarged sectional view showing a state of the device-side engager, the locking engager, and latch cover in a locked state of the drug injection device;
FIG. 11C is a partial sectional view of the drug injection device in the basic state;
FIG. 12 is a flowchart showing a self-injection process;
FIG. 13 is a flowchart of a cartridge attachment process;
FIG. 14 is a flowchart of a process including an automatic puncturing process and an automatic injection process;
FIG. 15 is a flowchart of a process including an automatic needle withdrawal process; and
FIG. 16 is a diagram showing an example of a variation of the drug injection device.

### Description of Embodiments

Hereinafter, the drug cartridge and the drug injection device according to the present invention will be described with reference to the drawings. The same reference numerals are given to the same components.

### [Embodiment]

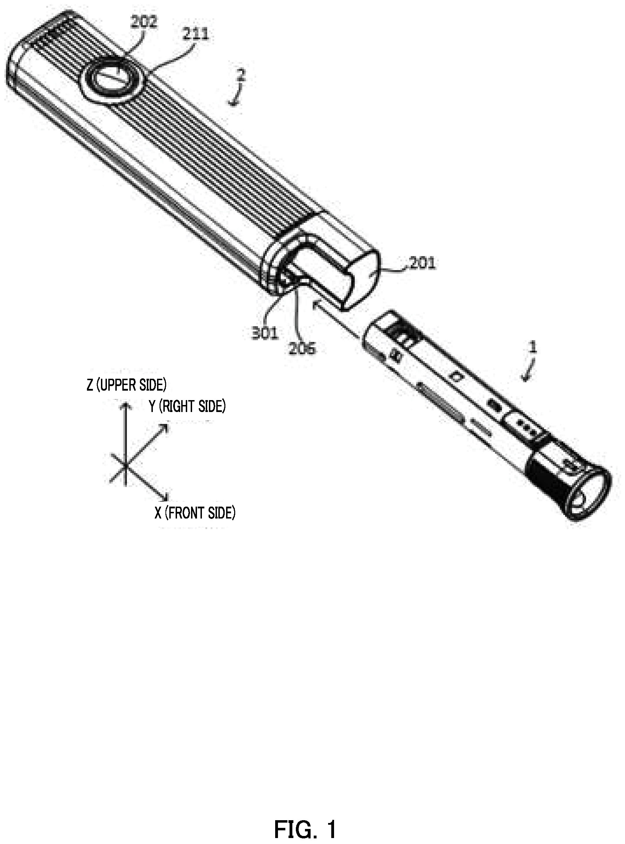

The drug cartridge and the drug injection device according to the embodiment of the present invention will be described with reference to FIGS. 1 to 15.

FIG. 1 is a perspective view of drug cartridge 1 and drug injection device 2 according to the embodiment of the present invention. Drug cartridge 1 and drug injection device 2 can be collectively regarded as a drug injection system.

Drug cartridge 1 and drug injection device 2 are used by the user to inject the drug into his/her body. Hereinafter, the act of the user injecting the drug into his/her body is referred to as self-injection. First, an outline of a usage aspect of drug cartridge 1 and drug injection device 2 will be described.

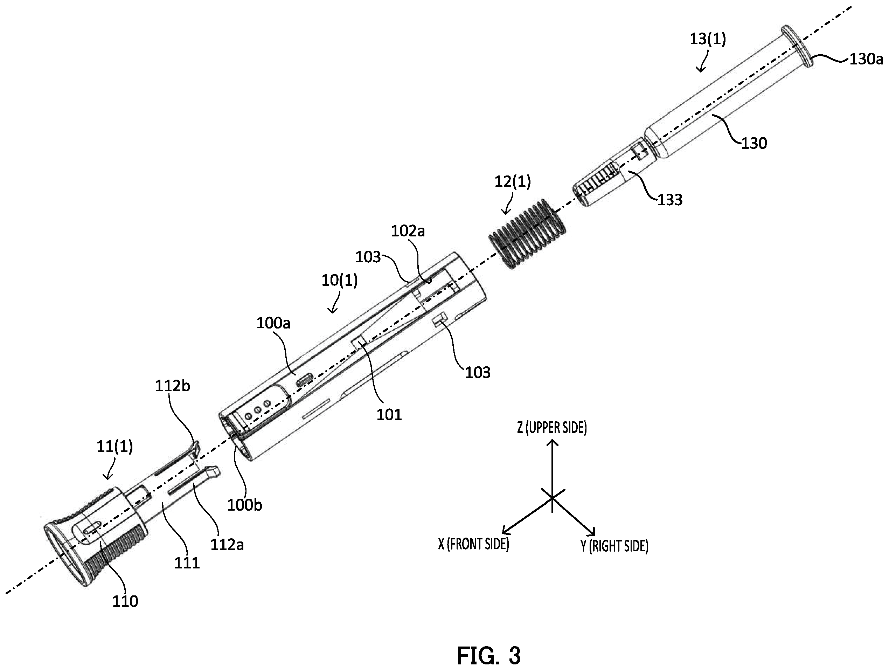

In a case of self-injection, the user attaches drug cartridge 1 to drug injection device 2. Drug cartridge 1 accommodates syringe 13 (see FIG. 3) filled with the drug.

When drug cartridge 1 is attached to drug injection device 2, drug injection device 2 locks drug cartridge 1 to drug injection device 2 such that drug cartridge 1 does not separate from drug injection device 2. This operation is referred to as a locking operation of drug injection device 2.

Next, the user brings skin detector 201 (see FIG. 1) provided at a distal end portion of drug injection device 2 into contact with his/her skin (that is, the skin). In this state, the user presses an injection button 202 of drug injection device 2.

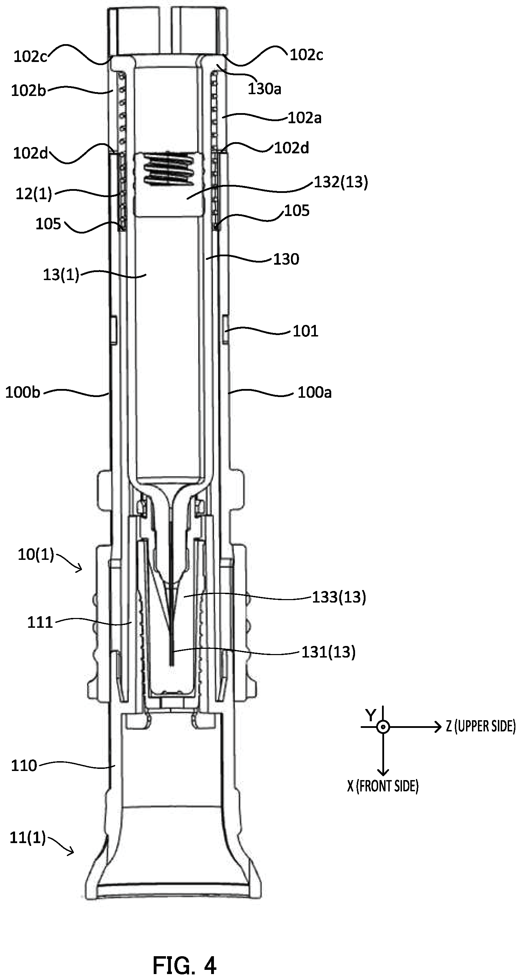

When injection button 202 is pressed in a state where skin detector 201 is in contact with the skin (that is, the skin) of the user, drug injection device 2 performs an operation of inserting needle 131 (see FIG. 4) of syringe 13 into the user. The operation of inserting needle 131 of syringe 13 into the user is referred to as a puncturing operation of drug injection device 2.

When needle 131 of syringe 13 is inserted into the user, drug injection device 2 performs an operation of injecting into the user the drug with which syringe 13 is filled. The operation of injecting into the user the drug with which syringe 13 is referred to as an injection operation of drug injection device 2.

When the injection operation ends, drug injection device 2 performs an operation of withdrawing needle 131 (see FIG. 4) of syringe 13 from the user. The operation of withdrawing needle 131 of syringe 13 from the user is referred to as a needle withdrawal operation of drug injection device 2.

Drug injection device 2 releases the locking of drug cartridge 1 to drug injection device 2 in the course of the needle withdrawal operation. The operation of releasing the locking of drug cartridge 1 to drug injection device 2 is referred to as an unlocking operation of drug injection device 2.

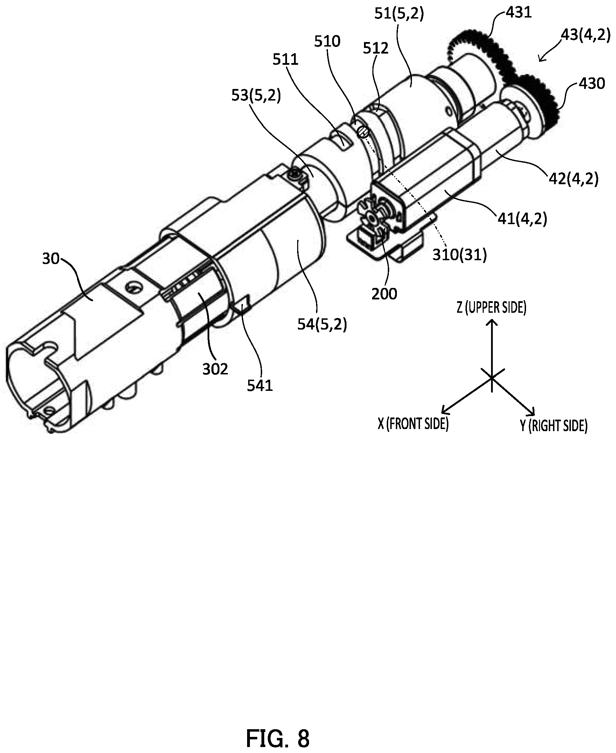

Drug injection device 2 having the above-described configuration performs the locking operation, the puncturing operation, the injection operation, the needle withdrawal operation, and the unlocking operation based on power of a motor 41 (see FIG. 8).

Hereinafter, specific configurations of drug cartridge 1 and drug injection device 2 will be described. In describing the structures of drug cartridge 1 and drug injection device 2, a rectangular coordinate system (X, Y, Z) shown in each of the figures is used.

The X-direction matches a front-rear direction of drug cartridge 1 and drug injection device 2. The positive X-direction matches a front side of drug cartridge 1 and drug injection device 2. The positive X-direction is also a direction in which syringe 13 moves in the puncturing operation of drug injection device 2. Therefore, the positive X-direction is also referred to as a puncturing direction.

The negative X-direction matches a rear side of drug cartridge 1 and drug injection device 2. The negative X-direction is also a direction in which syringe 13 moves in the needle withdrawal operation of drug injection device 2. Therefore, the negative X-direction is also referred to as a needle withdrawal direction.

The Y-direction matches a left-right direction and a width direction of drug cartridge 1 and drug injection device 2. The positive Y-direction matches a right side in a case of viewing drug cartridge 1 and drug injection device 2 from the front. The negative Y-direction matches a left side in a case of viewing drug cartridge 1 and drug injection device 2 from the front.

The Z-direction matches an up-down direction (thickness direction) of drug cartridge 1 and drug injection device 2. The positive Z-direction matches an upper side of drug cartridge 1 and drug injection device 2. The negative Z-direction matches a lower side of drug cartridge 1 and drug injection device 2.

### (Drug Cartridge)

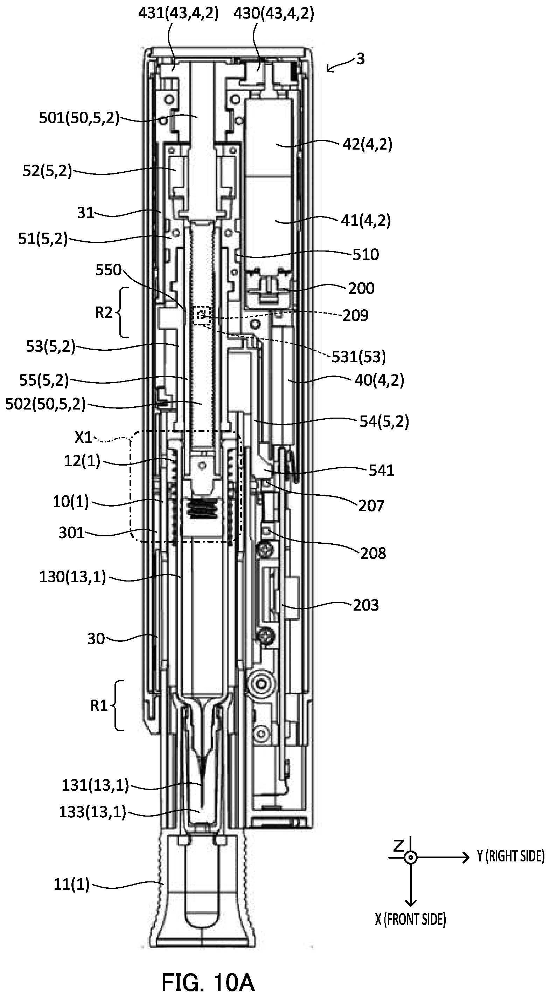

First, a specific configuration of drug cartridge 1 will be described with reference to FIGS. 3 to 7. Drug cartridge 1 is a disposable type cartridge that is replaced for each self-injection. Drug cartridge 1 is used in a state of being accommodated in cartridge housing 301 (see FIG. 10A) of drug injection device 2 described below in a case of self-injection.

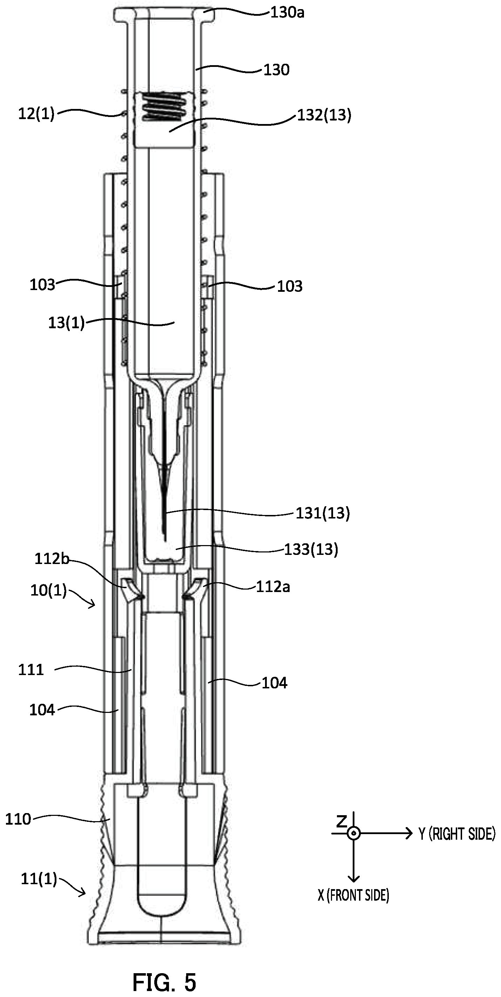

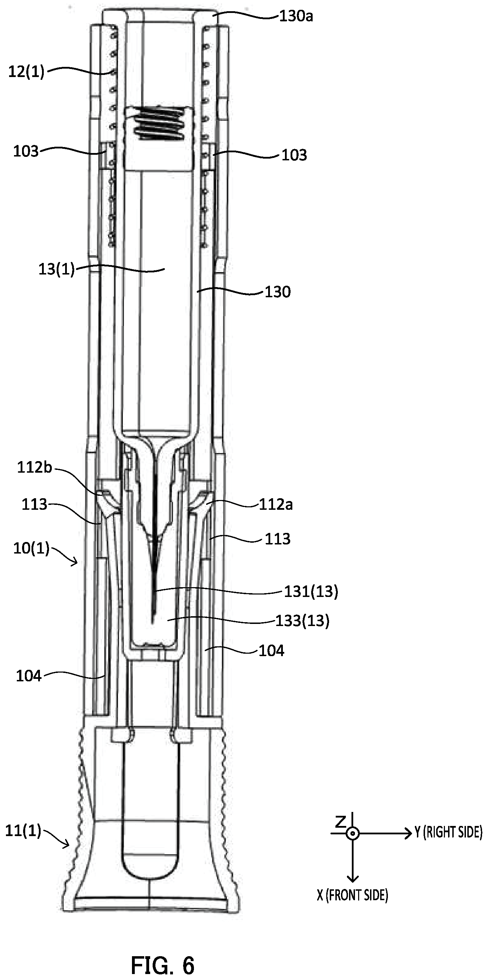

FIG. 3 is an exploded perspective view of drug cartridge 1. FIG. 4 is a sectional view of drug cartridge 1 cut along an XZ plane as viewed from the positive Y-direction. FIGS. 5 and 6 are sectional views showing a state in the course of an assembly step of drug cartridge 1.

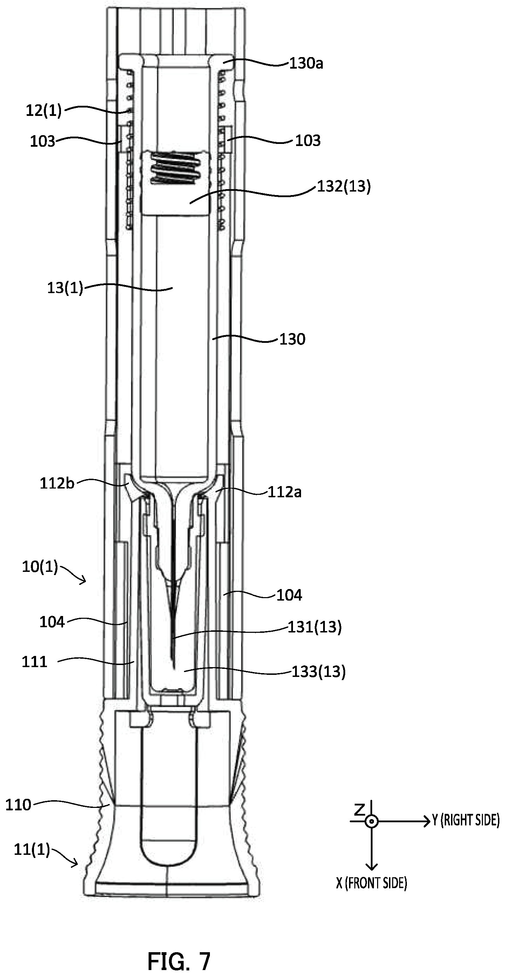

FIG. 7 is a sectional view of drug cartridge 1 cut along an XY plane as viewed from the positive Z-direction. Drug cartridge 1 shown in FIGS. 4 and 7 is drug cartridge 1 in a state before being attached to drug injection device 2.

The state of drug cartridge 1 shown in FIGS. 4 and 7 is referred to as a basic state of drug cartridge 1. In addition, positions of members constituting drug cartridge 1 in the basic state of drug cartridge 1 are referred to as basic positions of the members.

As shown in FIG. 3, drug cartridge 1 includes case 10, cap 11, spring 12, and syringe 13. Case 10, cap 11, spring 12, and syringe 13 are assembled in the state shown in FIGS. 4 and 7.

### (Case)

As shown in FIG. 3, case 10 has a cylindrical shape extending in the front-rear direction. Case 10 includes a pair of flat surface portions 100a and 100b facing each other. The pair of flat surface portions 100a and 100b face each other in the up-down direction.

Drug cartridge 1 can be accommodated in cartridge housing 301 of drug injection device 2 in a state where flat surface portion 100a of case 10 faces upward. In addition, drug cartridge 1 can be accommodated in cartridge housing 301 of drug injection device 2 also in a state where flat surface portion 100b of case 10 faces upward.

### (RFID Tag)

Case 10 includes RFID tag 101 on one flat surface portion 100a of the pair of flat surface portions 100a and 100b. RFID tag 101 may be provided on flat surface portion 100b.

RFID tag 101 can transmit and receive information by short-range communication using radio waves. RFID tag 101 may store information (for example, type and amount) related to the drug with which syringe 13 is filled. In addition, RFID tag 101 may store information such as a manufacturing date and/or an expiration date of syringe 13.

RFID tag 101 is covered with a seal member (not shown). RFID tag 101 may be of a type in which information can be added or information can be rewritten from the outside. The information stored in RFID tag 101 may be protected by an anti-tampering function.

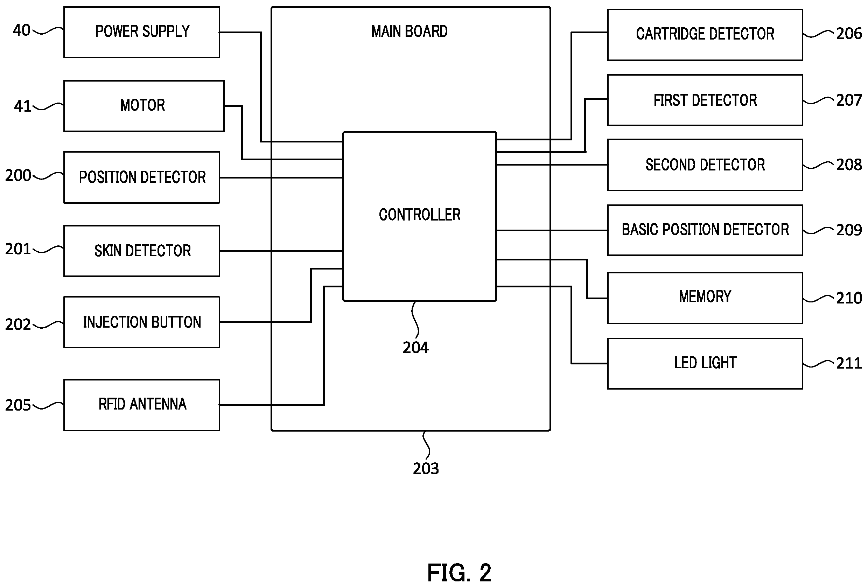

The information stored in RFID tag 101 is read by drug injection device 2 (specifically, an RFID antenna 205 (see FIG. 2)) in a state where drug cartridge 1 is attached to drug injection device 2. It is also possible to use an NFC tag instead of RFID tag 101.

Case 10 includes a pair of slits 102a and 102b (see FIGS. 3 and 4) extending in the front-rear direction in each of the pair of flat surface portions 100a and 100b. Slits 102a and 102b restrict a movement distance of syringe 13 in the front-rear direction. In other words, syringe 13 can move in the front-rear direction by a distance corresponding to a length of slits 102a and 102b in the front-rear direction.

Rear end portion 102c (see FIG. 4) of each of slits 102a and 102b engages with a rear end portion (specifically, flange 130a) of syringe 13 (specifically, syringe body 130) in the basic state of drug cartridge 1. Rear end portions 102c of slits 102a and 102b correspond to one example of a second case-side engager.

That is, slits 102a and 102b restrict the rearward movement of syringe 13 based on the engagement with syringe 13 in the basic state of drug cartridge 1.

In addition, slits 102a and 102b prevent syringe 13 from being pulled out rearward in the basic state of drug cartridge 1. That is, slits 102a and 102b also have a function of preventing syringe 13 from being pulled out.

In addition, front end portions 102d of slits 102a and 102b restrict the forward movement of syringe 13 based on the engagement with syringe 13. Front end portions 102d of slits 102a and 102b correspond to one example of a third case-side engager.

Specifically, when syringe 13 moves forward from the basic position shown in FIG. 4, flange 130a of syringe 13 comes into contact with front end portions 102d of slits 102a and 102b. Then, the forward movement of syringe 13 is restricted by front end portions 102d of slits 102a and 102b.

In addition, case 10 includes locking engagers 103 (see FIG. 3). Locking engagers 103 include a plurality (in the present embodiment, four) of through-holes penetrating case 10 in the radial direction.

In the present embodiment, locking engagers 103 are provided at positions spaced at equal intervals in the circumferential direction of case 10.

Locking engagers 103 are provided on a rear end portion side of drug cartridge 1 and at positions corresponding to front end portions of slits 102a and 102b in the front-rear direction. Positions of locking engagers 103 in the front-rear direction are the same as each other. Locking engagers 103 and the front end portions of slits 102a and 102b may not be provided at the corresponding positions.

Locking engagers 103 engage with holder 30 (specifically, device-side engagers 302) of drug injection device 2 in a state where drug cartridge 1 is attached to drug injection device 2 (hereinafter, also referred to as a "attached state of drug cartridge 1").

In addition, case 10 includes engagement retainer 104 at a front end portion on an inner peripheral surface. Engagement retainers 104 suppress release of the engagement between needle shield 133 of syringe 13 and cap 11 during an operation of removing cap 11 from case 10. Engagement retainers 104 correspond to one example of a radial expansion preventer.

Engagement retainers 104 include a plurality (in the present embodiment, four) of ribs provided on the inner peripheral surface of case 10. The plurality of ribs are spaced at equal intervals in the circumferential direction. In FIG. 7, some of the plurality of ribs are not shown. The function of engagement retainers 104 will be described below. The configuration of engagement retainers 104 is not limited to the ribs.

In addition, case 10 includes spring seat 105 (see FIG. 4) on the inner peripheral surface. Spring seats 105 correspond to one example of a first case-side engager. Spring seats 105 are formed by rear end surfaces of a plurality of ribs extending in the front-rear direction. Spring seats 105 are portions that come into contact with a front end portion of spring 12. The configuration of spring seats 105 is not limited to the ribs.

### (Cap)

Cap 11 is attached to a front end portion of case 10. Cap 11 has a tubular shape in which opposite end portions in the front-rear direction are open.

Specifically, cap 11 includes large-diameter tubular portion 110 and small-diameter tubular portion 111. Large-diameter tubular portion 110 is provided in a front half portion of cap 11. Small-diameter tubular portion 111 is provided in a rear half portion of cap 11. Large-diameter tubular portion 110 and small-diameter tubular portion 111 are connected by an annular stepped portion.

Cap 11 includes a pair of cap-side engagers 112a and 112b (see FIG. 3) on small-diameter tubular portion 111. Cap-side engagers 112a and 112b each have an arm shape extending in the front-rear direction. Cap-side engagers 112a and 112b face each other in the radial direction of small-diameter tubular portion 111.

Cap-side engagers 112a and 112b are elastically deformed in the radial direction of small-diameter tubular portion 111. When cap-side engagers 112a and 112b are elastically deformed in the radial direction of small-diameter tubular portion 111, a distance between cap-side engagers 112a and 112b changes.

Cap-side engagers 112a and 112b each include a claw portion at a rear end portion. The claw portion engages with needle shield 133 of syringe 13 in the basic state of drug cartridge 1.

### (Spring)

Spring 12 is a coil spring. Spring 12 is accommodated in case 10. In addition, syringe 13 is inserted through spring 12. A central axis of spring 12 matches a central axis of case 10 and syringe 13.

A rear end portion of spring 12 comes into contact with flange 130a of syringe 13 in the basic state of drug cartridge 1. A front end portion of spring 12 comes into contact with spring seat 105 (see FIG. 4) of case 10 in the basic state of drug cartridge 1.

Spring 12 having the above-described configuration is compressed between syringe 13 and case 10 in the basic state of drug cartridge 1. In this state, spring 12 constantly biases syringe 13 rearward.

### (Syringe)

Syringe 13 includes syringe body 130, needle 131, syringe-side piston 132, and needle shield 133.

Syringe body 130 has a tubular shape and includes a housing configured to accommodate the drug. Syringe body 130 includes flange 130a at the rear end portion. Flange 130a corresponds to one example of a syringe-side engager.

Flange 130a has an annular shape that extends from an outer peripheral surface of syringe body 130 to an outer side in the radial direction of syringe body 130.

The rear end surface of flange 130a engages with rear end portions 102c of slits 102a and 102b in case 10 in the basic state of drug cartridge 1. The front end surface of flange 130a comes into contact with a rear end portion of spring 12 in the basic state of drug cartridge 1.

Flange 130a is constantly biased rearward by spring 12. The rearward movement of flange 130a is restricted by rear end portions 102c of slits 102a and 102b in case 10.

Needle 131 is provided at the front end portion of syringe body 130. Needle shield 133 is attached to the front end portion of syringe body 130 to cover needle 131.

Syringe-side piston 132 is accommodated in the housing of syringe body 130. Syringe-side piston 132 can move in the front-rear direction in syringe body 130. The housing of syringe body 130 is filled with the drug in front of syringe-side piston 132.

When syringe-side piston 132 moves forward in a state where needle shield 133 is removed from syringe body 130, the drug present on the front side of syringe-side piston 132 in syringe body 130 moves forward. Then, the drug is injected to the outside from a distal end portion of needle 131.

### (Method Of Assembling Drug Cartridge)

Next, a method of assembling drug cartridge 1 will be described with reference to FIGS. 5 to 7. The method of assembling drug cartridge 1 may be performed by a manual operation of an operator or may be performed by an assembly device.

Hereinafter, a procedure performed when the operator performs the method of assembling drug cartridge 1 will be described. When the method of assembling drug cartridge 1 is performed by the assembly device, the following description may be appropriately replaced.

First, as shown in FIG. 5, the operator places cap 11 on the front end portion of case 10. In this state, cap 11 is not fixed to case 10. More specifically, cap 11 is press-fitted and temporarily fixed to engagement retainer 104 of case 10 with a light force by small-diameter tubular portion 111.

Next, as shown in FIG. 5, the operator inserts spring 12 into case 10 from an opening portion in a rear side of case 10. In this state, spring 12 is in a free state. In addition, the rear end portion of spring 12 is positioned rearward of the rear end portion of case 10. Front end portion of spring 12 comes into contact with spring seat 105 (see FIG. 4).

Although not shown, in the state shown in FIG. 5, the rear end portion of spring 12 may engage with rear end portions 102c of slits 102a and 102b in case 10. In this state, spring 12 may be compressed. This configuration contributes to improving the efficiency of the assembly operation of drug cartridge 1.

Next, as shown in FIG. 5, the operator inserts syringe 13 into case 10 from the opening portion in the rear side of case 10. In this case, syringe 13 is inserted into spring 12 from the rear side. In this state, the operator supports cap 11 from below such that cap 11 does not separate from case 10.

Then, the operator moves syringe 13 downward from the position shown in FIG. 5. Syringe 13 moves downward while contracting spring 12. As a result, syringe 13 moves to the position shown in FIG. 6.

Next, the operator further moves syringe 13 downward from the state shown in FIG. 6. Then, flange 130a of syringe 13 is press-fitted to an inner peripheral surface of the rear end portion of case 10 while syringe 13 moves downward.

The operator moves syringe 13 to the position (that is, the basic position) shown in FIG. 7. As a result, flange 130a of syringe 13 engages with slits 102a and 102b of case 10. Thus, the assembly of drug cartridge 1 is completed.

When syringe 13 moves from the position shown in FIG. 5 to the position shown in FIG. 6, needle shield 133 of syringe 13 is inserted into small-diameter tubular portion 111 of cap 11 from the rear side. In this case, as shown in FIG. 6, needle shield 133 is inserted into small-diameter tubular portion 111 while spreading cap-side engagers 112a and 112b.

In other words, when needle shield 133 enters small-diameter tubular portion 111 of cap 11, cap-side engagers 112a and 112b are elastically deformed outward in the radial direction of small-diameter tubular portion 111.

As described above, in the present embodiment, when needle shield 133 enters small-diameter tubular portion 111 of cap 11, cap-side engagers 112a and 112b are expanded to allow needle shield 133 to enter small-diameter tubular portion 111.

Specifically, in a state where cap 11 is assembled to the front end portion of case 10, radial-expansion-allowing space 113 (see FIG. 6) configured to allow cap-side engagers 112a and 112b to expand is present between cap-side engagers 112a and 112b and an inner surface of case 10.

As described above, in the present embodiment, since radial-expansion-allowing space 113 is present, needle shield 133 can enter small-diameter tubular portion 111 of cap 11. Radial-expansion-allowing space 113 corresponds to an example of a radial expansion allowing section.

When needle shield 133 proceeds to the position shown in FIG. 7, cap-side engagers 112a and 112b are elastically deformed to be reduced in diameter, and the claw portions of cap-side engagers 112a and 112b engage with a rear end portion of needle shield 133.

In the basic state shown in FIG. 7, needle shield 133 and cap-side engagers 112a and 112b are engaged with each other with an engagement force larger than a force that would separate cap 11 from case 10 under gravity.

The engagement force means a force required for the user to pull cap 11 downward to separate cap 11 from case 10 in the basic state shown in FIG. 7.

In the present embodiment, the engagement force between cap-side engagers 112a and 112b and needle shield 133 in the basic state shown in FIG. 7 is set to be larger than the spring force of spring 12.

That is, in the basic state shown in FIG. 7, when the user pulls cap 11 downward, spring 12 is contracted in a state where the engagement between cap-side engagers 112a and 112b and needle shield 133 is maintained.

Therefore, in the basic state shown in FIG. 7, when cap 11 is pulled downward, entire syringe 13 moves forward in a state where the engagement between cap-side engagers 112a and 112b and needle shield 133 is maintained.

Here, an operation of drug cartridge 1 when cap 11 is removed from case 10 from the basic state shown in FIG. 7 will be described. When cap 11 is removed from case 10 for self-injection, drug cartridge 1 is attached to drug injection device 2.

The user pulls cap 11 forward from the basic state shown in FIG. 7. Then, cap 11 moves forward. Since cap-side engagers 112a and 112b of cap 11 are engaged with needle shield 133, needle shield 133 also moves forward.

The engagement force between cap-side engagers 112a and 112b and needle shield 133 in the basic state shown in FIG. 7 is set to be larger than the spring force of spring 12. Therefore, when cap 11 moves forward, spring 12 is contracted, and entire syringe 13 moves forward together with cap 11.

Then, syringe 13 moves to a position where flange 130a comes into contact with front end portions 102d of slits 102a and 102b. Although not shown, this state is referred to as a first state of syringe 13 and cap 11. In addition, a position of syringe 13 and cap 11 in the first state is referred to as a first position of syringe 13 and cap 11.

In the first state of syringe 13 and cap 11, cap-side engagers 112a and 112b are positioned inside engagement retainer 104 of case 10. In this state, cap-side engagers 112a and 112b are suppressed from being radially expanded by engagement retainer 104.

In other words, in the first state of syringe 13 and cap 11, cap-side engagers 112a and 112b cannot be so expanded radially as to release the engagement between needle shield 133 and cap-side engagers 112a and 112b.

The user further pulls cap 11 forward from the first state of syringe 13 and cap 11. Then, cap 11 moves forward in a state where the radial expansion of cap-side engagers 112a and 112b is suppressed by engagement retainer 104.

In this state, the movement of syringe body 130 forward is restricted by front end portions 102d of slits 102a and 102b, so that syringe body 130 cannot move forward. Therefore, needle shield 133 separates from syringe body 130.

Then, needle shield 133 moves forward together with cap 11, and cap 11 and needle shield 133 separate from drug cartridge 1. When needle shield 133 separates from syringe body 130, syringe body 130 moves rearward based on the biasing force of spring 12 and returns to the basic position shown in FIG. 7.

In a state where cap 11 is separated from syringe body 130, needle 131 is accommodated in case 10 as shown in FIG. 7. In other words, in the state shown in FIG. 7, needle 131 is not exposed to the outside from the distal end portion of case 10. This configuration contributes to improving safety.

### (Drug Injection Device)

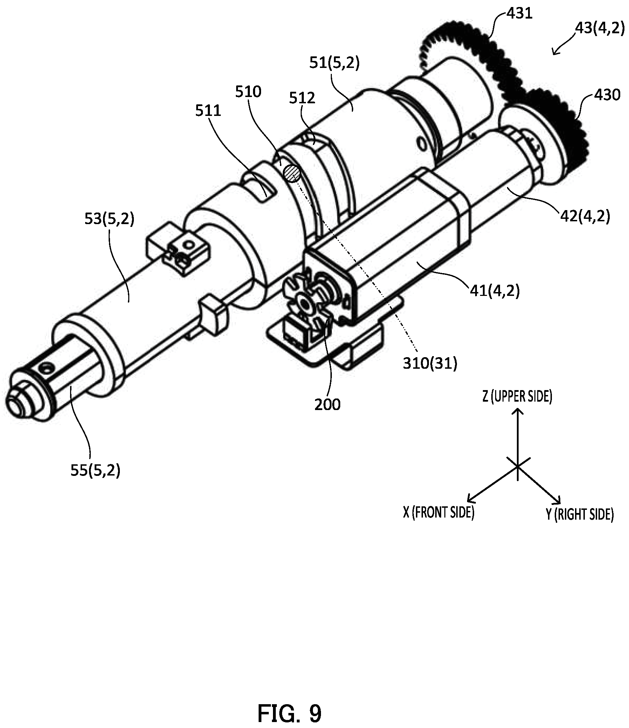

Next, a configuration of drug injection device 2 will be described with reference to FIGS. 8 to 15. FIGS. 8 and 9 are perspective views of only a part of the configuration of drug injection device 2. FIGS. 10A to 10F are sectional views of drug injection device 2 taken along the XY plane as viewed from the positive Z-direction.

Specifically, FIG. 10A is a sectional view of drug injection device 2 in the basic state. In FIG. 10A, drug cartridge 1 is inserted into drug injection device 2. However, drug cartridge 1 is not locked to drug injection device 2. The state of drug injection device 2 shown in FIG. 10A is also referred to as a non-locked state.

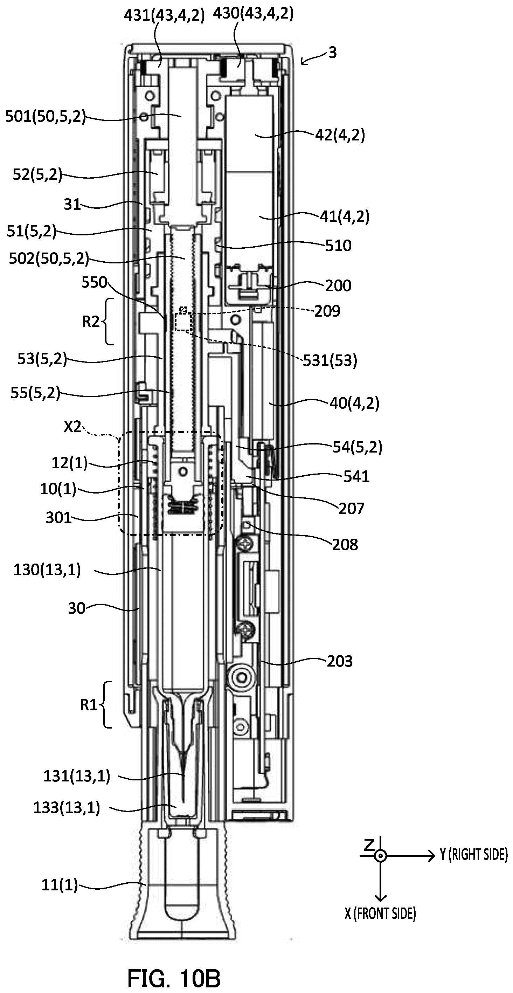

In addition, FIG. 10B is a sectional view of drug injection device 2 after the locking operation. In other words, FIG. 10B is a sectional view showing the locked state of drug injection device 2. In the state shown in FIG. 10B, drug cartridge 1 is locked to drug injection device 2.

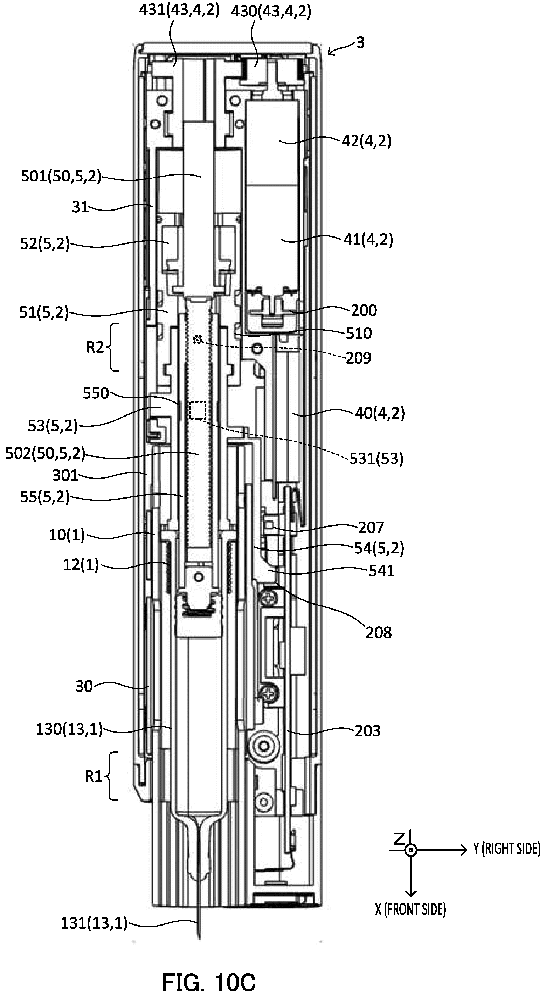

In addition, FIG. 10C is a sectional view of drug injection device 2 after the puncturing operation. In other words, FIG. 10C is a sectional view showing a puncture completion state of drug injection device 2.

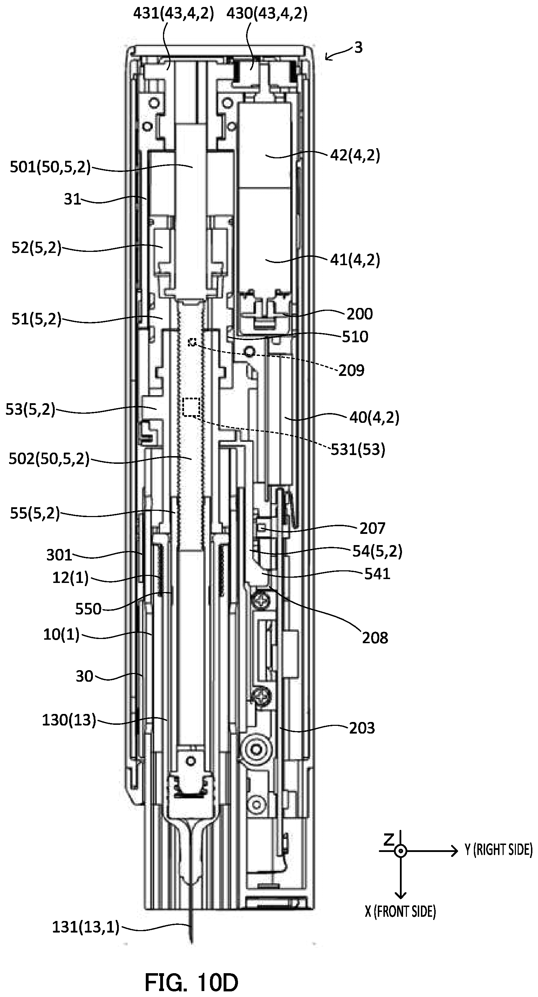

In addition, FIG. 10D is a sectional view of drug injection device 2 after the injection operation. In other words, FIG. 10D is a sectional view showing an injection completion state of drug injection device 2.

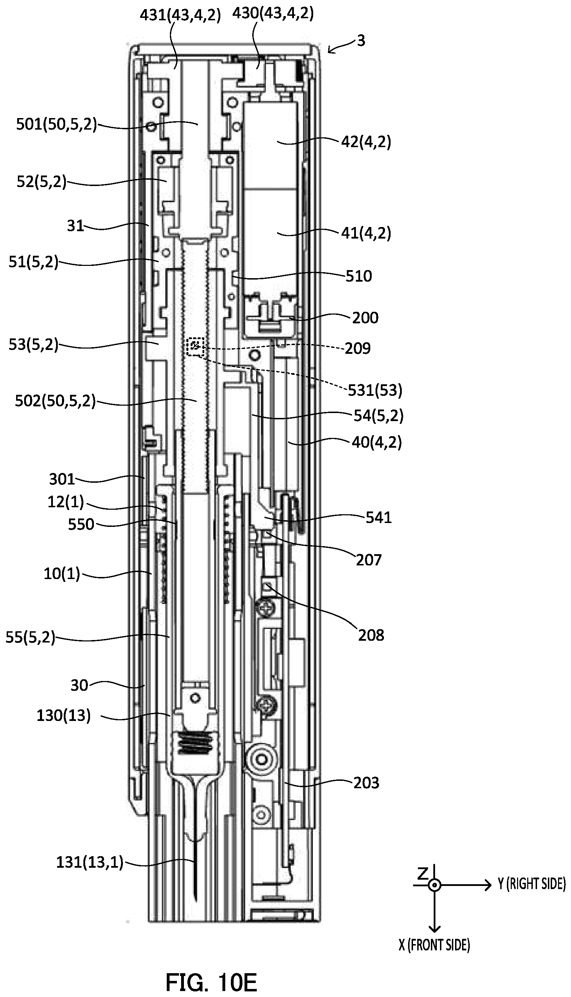

In addition, FIG. 10E is a sectional view of a state (in other words, immediately after the needle withdrawal) in which the needle withdrawal of drug injection device 2 is completed. The state of drug injection device 2 shown in FIG. 10E is different from the state of drug injection device 2 shown in FIG. 10A in that piston 55 is not fully returned.

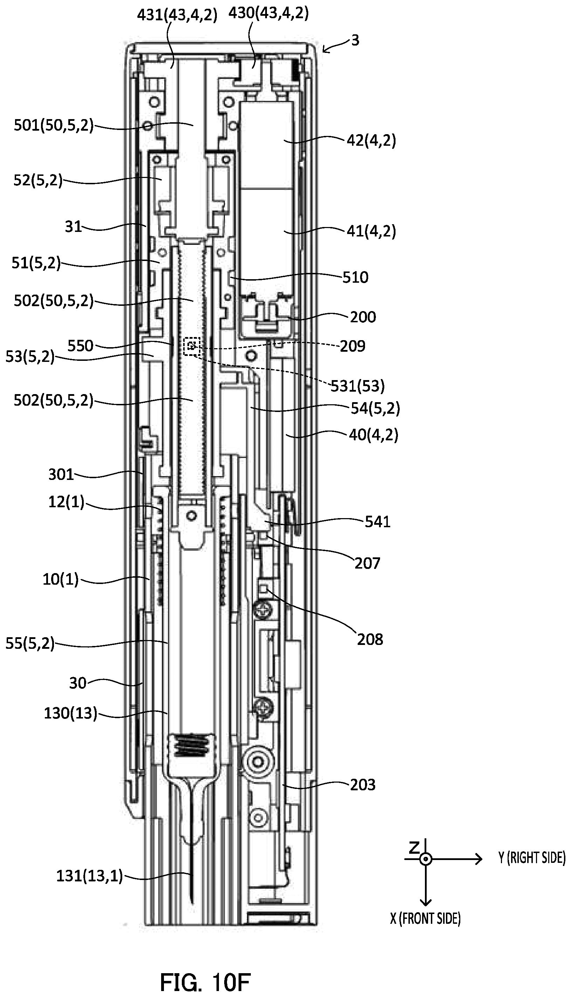

In addition, FIG. 10F is a sectional view of a state in which all the needle withdrawal operations of drug injection device 2 are completed. The state of drug injection device 2 shown in FIG. 10F is the same basic state as the state of drug injection device 2 shown in FIG. 10A.

Drug injection device 2 performs various operations in a case of self-injection. Specifically, drug injection device 2 performs the locking operation, the puncturing operation, the injection operation, the needle withdrawal operation, and the unlocking operation.

The locking operation is an operation of locking drug cartridge 1. The puncturing operation is an operation of inserting needle 131 of syringe 13 into the user. The injection operation is an operation of injecting the drug in syringe 13 into the user.

The needle withdrawal operation is an operation of withdrawing needle 131 of syringe 13 from the user. The unlocking operation is an operation of releasing the locking of drug cartridge 1.

In addition, drug injection device 2 can take a state corresponding to each of the above-described operations. A state in which drug cartridge 1 is not attached to drug injection device 2 is referred to as a basic state of drug injection device 2. A state in which drug cartridge 1 is attached to drug injection device 2 and drug cartridge 1 is not locked to drug injection device 2 (that is, the state shown in FIG. 10A) is also the basic state of drug injection device 2.

In addition, a state of drug injection device 2 after the puncturing operation and before the injection operation is referred to as a puncture completion state of drug injection device 2. In addition, a state of drug injection device 2 after the injection operation and before the needle withdrawal operation is referred to as an injection completion state of drug injection device 2.

In addition, a state of drug injection device 2 after the needle withdrawal operation (the state shown in FIG. 10E) is referred to as a needle withdrawal completion state of drug injection device 2.

Drug injection device 2 mainly includes casing 3, driver 4, and actuator 5.

### (Casing)

Casing 3 accommodates elements constituting drug injection device 2. Casing 3 includes holder 30 (see FIGS. 8 and 10A) that holds drug cartridge 1. Holder 30 corresponds to an example of a first locking member and has a tubular shape. Holder 30 is provided on a left half portion of casing 3.

A space surrounded by holder 30 constitutes a part of cartridge housing 301 in which drug cartridge 1 is accommodated. Cartridge housing 301 is a tubular space extending in the front-rear direction.

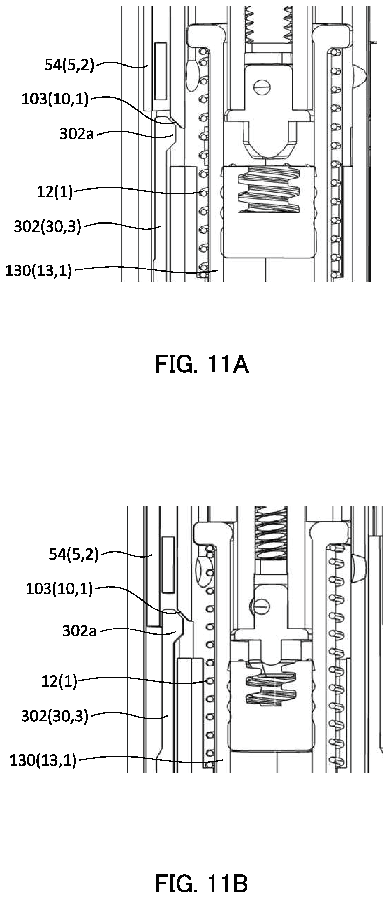

Holder 30 includes device-side engagers 302 (see FIGS. 8, 11A, and 11B) that engage with locking engagers 103 (see FIGS. 3, 11A, and 11B) of drug cartridge 1. The number of device-side engagers 302 is the same as the number of locking engagers 103.

In a state where drug cartridge 1 is attached to drug injection device 2, device-side engagers 302 engage with locking engagers 103 (see FIG. 3) from the outside of drug cartridge 1 (specifically, the outer side in the radial direction).

In addition, casing 3 includes inner case element 31. Inner case element 31 has a tubular shape. Slider 51 (see FIG. 10A) described below is inserted into inner case element 31. In other words, inner case element 31 is disposed to cover an outer peripheral surface of slider 51.

Inner case element 31 includes guide protrusion 310 (see FIGS. 8 and 9) on the inner peripheral surface. In FIGS. 8 and 9, inner case element 31 is not shown. In FIGS. 8 and 9, guide protrusion 310 is indicated by a black circle with hatching.

Guide protrusion 310 protrudes from the inner peripheral surface of inner case element 31 to an inner side of inner case element 31 in the radial direction. Guide protrusion 310 is always inserted into a helical guide groove 510 (see FIGS. 8 and 9) provided in slider 51.

When slider 51 is rotated, guide groove 510 of slider 51 is guided by guide protrusion 310, and slider 51 moves in the front-rear direction.

In the basic state of drug injection device 2, guide protrusion 310 is positioned at start point 511 (that is, front end portion) of guide groove 510. In the basic state of drug injection device 2, a position of guide protrusion 310 in guide groove 510 is referred to as a start point position of guide protrusion 310.

In the locked state of drug injection device 2, guide protrusion 310 is positioned at a position slightly moved from start point 511 (that is, front end portion) of guide groove 510 toward end point 512 (hereinafter, referred to as a "locked position"). In the locked state of drug injection device 2, the position of guide protrusion 310 in guide groove 510 is referred to as a locked position of guide protrusion 310.

In the puncture completion state of drug injection device 2, guide protrusion 310 is positioned at end point 512 (that is, the rear end portion) of guide groove 510. In the puncture completion state of drug injection device 2, the position of guide protrusion 310 in guide groove 510 is referred to as an end point position of guide protrusion 310.

In practice, guide protrusion 310 does not move in the front-rear direction. A positional relationship between guide protrusion 310 and guide groove 510 relatively changes as slider 51 moves with respect to inner case element 31.

As described above, inner case element 31 (in other words, casing 3) may be regarded as a member that guides the movement of slider 51 in the front-rear direction.

Cartridge housing 301 has an opening portion at front end portion. Drug cartridge 1 is inserted into cartridge housing 301 from the opening portion of cartridge housing 301. The opening portion of cartridge housing 301 may be, for example, closed by a lid member (not shown) in a situation where drug cartridge 1 is not inserted.

Casing 3 may be composed of a single member or may be composed of a plurality of members combined with each other. In addition, in the following description, the concept of "supported by casing 3" includes not only a configuration directly supported by casing 3 but also a configuration indirectly supported by casing 3 via another member.

### (Driver)

Driver 4 drives actuator 5 described below. Driver 4 is disposed in a right half portion of casing 3. The right half portion of casing 3 is referred to as a first housing. On the other hand, the left half portion of casing 3 is referred to as a second housing.

Driver 4 includes power supply 40, motor 41, decelerator 42, and transmitter 43.

### (Power Supply)

Power supply 40 is supported by casing 3. Power supply 40 is, for example, a rechargeable lithium ion battery. Power supply 40 supplies power to the elements constituting drug injection device 2. Power supply 40 is disposed at an intermediate portion of the first housing in the front-rear direction.

### (Motor)

Motor 41 is supported by casing 3. Motor 41 is, for example, a DC motor. Motor 41 is composed of a single motor. That is, drug injection device 2 includes only one motor 41 as a drive source. This configuration contributes to the reduction in size of drug injection device 2.

Motor 41 is actuated under the control of controller 204 based on the power supplied from power supply 40. The output (that is, the rotation) of motor 41 is transmitted to decelerator 42 described below. The rotational direction of motor 41 is controlled by controller 204. Motor 41 is disposed rearward of power supply 40 in the first housing.

Position detector 200 is provided in motor 41. Position detector 200 is, for example, a rotary encoder. Position detector 200 is provided on a rotation shaft of motor 41 or a member that rotates with the rotation shaft.

Position detector 200 transmits a detection value to controller 204. Controller 204 acquires information (specifically, the rotational direction and the rotation amount of motor 41) on the actuation state of motor 41 based on the detection value of position detector 200.

Then, controller 204 acquires information on the state of actuator 5 based on the acquired information on the actuation state of motor 41. The information on the state of actuator 5 may include information on the position of each element constituting actuator 5 and/or information on the movement distance.

### (Decelerator)

Decelerator 42 is supported by casing 3. Decelerator 42 decelerates the output (that is, the rotation) of motor 41 and transmits the decelerated output to transmitter 43 described below. Decelerator 42 is composed of, for example, a planetary gear mechanism. Decelerator 42 is not limited to the planetary gear mechanism and may be various types of speed reducers.

Decelerator 42 is disposed on the rear side of motor 41 in the first housing. Decelerator 42 and motor 41 are disposed on the same straight line parallel to the front-rear direction.

### (Transmitter)

Transmitter 43 is supported by casing 3. Transmitter 43 is connected to decelerator 42. Transmitter 43 transmits the power (that is, the rotation) of motor 41 transmitted from decelerator 42 to actuator 5 (specifically, feed screw 50) described below. Transmitter 43 includes first rotational transmission member 430 and second rotational transmission member 431.

Each of first rotational transmission member 430 and second rotational transmission member 431 has a tooth portion on an outer peripheral surface. First rotational transmission member 430 is disposed at a rear end portion of the first housing. First rotational transmission member 430 is fixed to an output shaft of decelerator 42. Therefore, first rotational transmission member 430 rotates based on the power transmitted from decelerator 42.

Second rotational transmission member 431 is disposed at a rear end portion of the second housing. The tooth portion of second rotational transmission member 431 and the tooth portion of first rotational transmission member 430 are meshed with each other. Second rotational transmission member 431 is connected to actuator 5 (specifically, feed screw 50) described below.

Each of first rotational transmission member 430 and second rotational transmission member 431 rotates about a central axis parallel to the front-rear direction. First rotational transmission member 430 and second rotational transmission member 431 are prevented from moving in the front-rear direction.

When first rotational transmission member 430 rotates based on the power (that is, the rotation) transmitted from decelerator 42, second rotational transmission member 431 rotates in synchronization with first rotational transmission member 430. When second rotational transmission member 431 rotates, actuator 5 is actuated.

### (Actuator)

Next, a configuration of actuator 5 will be described. Actuator 5 operates based on the power (that is, the rotation) transmitted from driver 4. Specifically, actuator 5 performs the locking operation, the puncturing operation, the injection operation, the needle withdrawal operation, and the unlocking operation based on the power of driver 4. The operations of actuator 5 will be described below.

Actuator 5 is disposed in the second housing of casing 3. The second housing is the left half portion of casing 3 as described above.

Actuator 5 mainly includes feed screw 50, slider 51, torque limiter 52, latch 53, latch cover 54, and piston 55.

### (Feed Screw)

Feed screw 50 is supported by casing 3. Feed screw 50 is a shaft-shaped member. Feed screw 50 is disposed in parallel to the front-rear direction. Feed screw 50 can rotate about a central axis parallel to the front-rear direction as a rotation center.

In addition, feed screw 50 can move in the front-rear direction. Feed screw 50 moves in the front-rear direction in conjunction with the movement of slider 51 in the front-rear direction. That is, feed screw 50 moves in synchronization with slider 51. A movement amount of feed screw 50 in the front-rear direction is the same as a movement amount of slider 51 in the front-rear direction.

Feed screw 50 is connected to driver 4. Specifically, feed screw 50 includes base portion 501 and screw portion 502. A front end portion of base portion 501 is connected to a rear end portion of screw portion 502. Screw portion 502 has a male thread portion on an outer peripheral surface.

Feed screw 50 is connected to second rotational transmission member 431 in transmitter 43 of driver 4. Specifically, the rear end portion of base portion 501 is inserted into second rotational transmission member 431.

The rear end portion of feed screw 50 (specifically, base portion 501) is composed of, for example, a D-cut portion having a D-shaped cross-sectional shape. In addition, an inner peripheral surface of second rotational transmission member 431 has a shape along an outer peripheral surface of the rear end portion of feed screw 50. Then, the rear end portion of feed screw 50 and second rotational transmission member 431 are engaged with each other in a state in which torque (in other words, rotation) can be transmitted.

In addition, the front end portion (specifically, screw portion 502) of feed screw 50 is connected to piston 55 described below. The male thread portion of screw portion 502 and piston 55 are screwed to each other.

Feed screw 50 having such a configuration rotates based on the rotation of second rotational transmission member 431. That is, the rotation of second rotational transmission member 431 is transmitted to feed screw 50.

In addition, feed screw 50 moves in the front-rear direction in conjunction with the movement of slider 51 in the front-rear direction. The operation of feed screw 50 will be described below.

### (Slider and Torque Limiter)

Slider 51 is supported by inner case element 31 of casing 3. Slider 51 corresponds to an example of a first moving member. As shown in FIG. 8, slider 51 has a tubular shape. Slider 51 is disposed in a state where the central axis is parallel to the front-rear direction.

Feed screw 50 is inserted into slider 51. The central axis of slider 51 and the central axis of feed screw 50 are disposed on the same straight line.

Slider 51 has helical guide groove 510 (see FIGS. 8 and 9) on the outer peripheral surface. Guide groove 510 extends from start point 511, which is front end portion, to end point 512, which is the rear end portion, along the outer peripheral surface of slider 51.

Guide groove 510 is engaged with guide protrusion 310 (see FIGS. 8 and 9) of inner case element 31 in casing 3.

In FIGS. 8 and 9, guide protrusion 310 is indicated by a black circle with hatching. In FIGS. 8 and 9, inner case element 31 is not shown.

Slider 51 moves in the front-rear direction in accordance with the rotation of slider 51. Slider 51 moves in the front-rear direction by being guided by guide protrusion 310 via guide groove 510. A movement amount of slider 51 in the front-rear direction (in other words, a stroke) is equal to a distance between start point 511 and end point 512 in the front-rear direction of guide groove 510.

In a state where slider 51 is positioned at the rear end of the stroke, guide protrusion 310 is positioned at start point 511 (that is, front end portion) of guide groove 510. On the other hand, in a state where slider 51 is positioned at the front end of the stroke, guide protrusion 310 is positioned at end point 512 (that is, the rear end portion) of guide groove 510.

Slider 51 is connected to feed screw 50 via torque limiter 52. That is, torque limiter 52 is provided between feed screw 50 and slider 51. The central axis of slider 51 and the central axis of torque limiter 52 are disposed on the same straight line.

Torque limiter 52 switches between a state in which the rotation of feed screw 50 is transmitted to slider 51 (hereinafter, also referred to as a "transmission state of the torque limiter") and a state in which the rotation of feed screw 50 is not transmitted to slider 51 (hereinafter, also referred to as a "non-transmission state of the torque limiter").

Torque limiter 52 corresponds to an example of a switching mechanism for switching between the puncturing operation of drug injection device 2 and the injection operation of drug injection device 2. The puncturing operation and the injection operation of drug injection device 2 will be described below.

Specifically, when the torque acting on torque limiter 52 is equal to or less than a predetermined value, torque limiter 52 is in the transmission state of the torque limiter. On the other hand, when the torque acting on torque limiter 52 is larger than the predetermined value, torque limiter 52 is in the non-transmission state of the torque limiter.

In the present embodiment, the torque acting on torque limiter 52 may be regarded as a torque acting on torque limiter 52 from feed screw 50.

A configuration of torque limiter 52 may be substantially the same as a configuration of a torque limiter that is generally known. Therefore, a description of a specific configuration of torque limiter 52 will be omitted.

Torque limiter 52 has a cylindrical shape. Feed screw 50 inserted extends through the interior defined by an inner peripheral surface of torque limiter 52. In addition, torque limiter 52 and feed screw 50 are engaged with each other in a state in which power (that is, rotation) can be transmitted.

In addition, torque limiter 52 and feed screw 50 are engaged with each other in a state in which torque limiter 52 and feed screw 50 can move in the front-rear direction in synchronization with each other. That is, torque limiter 52 and feed screw 50 move in the front-rear direction at the same time.

The outer peripheral surface of torque limiter 52 is fitted to the inner peripheral surface of slider 51. In other words, slider 51 is provided to cover the outer peripheral surface of torque limiter 52.

Torque limiter 52 and slider 51 are engaged with each other in a state in which power (that is, rotation) can be transmitted. In addition, torque limiter 52 and slider 51 are engaged with each other in a state in which torque limiter 52 and slider 51 can move in the front-rear direction in synchronization with each other. That is, torque limiter 52 and slider 51 move in the front-rear direction at the same time. The operation of slider 51 and torque limiter 52 will be described below.

### (Latch)

As shown in FIG. 9, latch 53 has a tubular shape. Latch 53 is connected to the front end portion of slider 51. Feed screw 50 and piston 55 are inserted into latch 53.

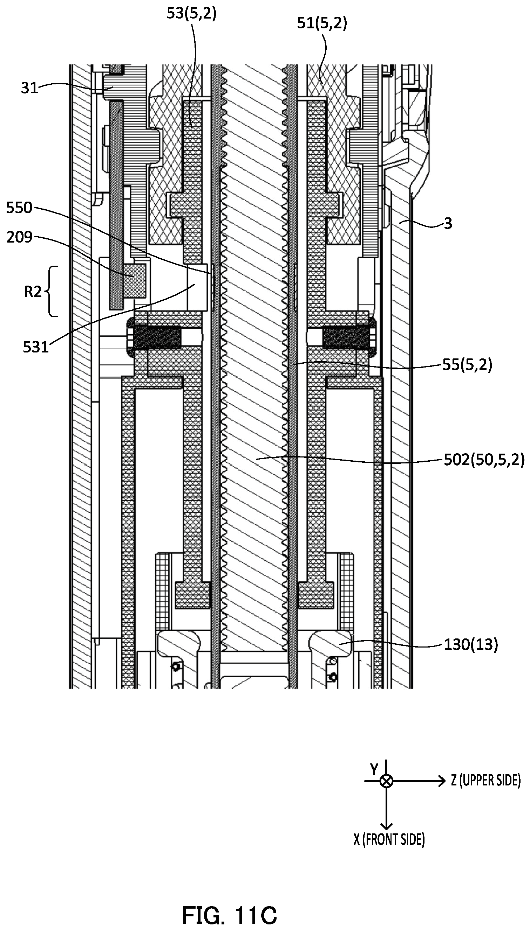

Latch 53 has through-hole 531 (see FIG. 11C) at a predetermined position in the front-rear direction. Through-hole 531 penetrates latch 53 in the radial direction of latch 53. Through-hole 531 is provided in a side surface of latch 53 on a negative Z-direction in an outer surface.

In the basic state (the state shown in FIGS. 10A, 10F, and 11C) of drug injection device 2, through-hole 531 faces detection target 550 (see FIGS. 10A, 10F, and 11C) of piston 55 in the radial direction of latch 53.

In addition, in the basic state (the state shown in FIGS. 10A, 10F, and 11C) of drug injection device 2, through-hole 531 faces basic position detector 209 (described below) in the radial direction of latch 53.

Latch 53 can move in the front-rear direction together with slider 51. However, the rotation of latch 53 is restricted based on the engagement with casing 3. Therefore, latch 53 moves in the front-rear direction together with slider 51 without rotating.

Specifically, a rear end portion of latch 53 is inserted into front end portion of slider 51. In other words, the end portion of latch 53 is covered by the front end portion of slider 51.

An outer peripheral surface of the rear end portion of latch 53 is engaged with an inner peripheral surface of front end portion of slider 51. Therefore, when slider 51 moves in the front-rear direction, latch 53 moves together with slider 51 based on the engagement between latch 53 and slider 51 in the front-rear direction.

The front end portion of latch 53 comes into contact with the rear end portion of syringe 13 (specifically, syringe body 130) in a state where drug cartridge 1 is attached to drug injection device 2 (see FIG. 10B).

In the puncturing operation of drug injection device 2, when latch 53 moves forward, latch 53 pushes syringe 13 (specifically, syringe body 130) forward. That is, latch 53 is a member configured to move syringe 13 (specifically, syringe body 130) forward in a case of the puncturing operation of drug injection device 2.

As a result of slider 51 moving forward by motor 41 in this way, syringe 13 moves forward. From such a viewpoint, slider 51 can be regarded as an example of the first moving member. In addition, when slider 51 moves forward by motor 41, slider 51 and latch 53 move forward. As a result, syringe 13 moves forward. From such a viewpoint, latch 53 can also be regarded as an example of the first moving member. Alternatively, a member obtained by combining slider 51 and latch 53 can also be regarded as an example of the first moving member.

### (Latch Cover)

Latch cover 54 has a tubular shape as shown in FIG. 8. Latch cover 54 corresponds to an example of a second locking member. Feed screw 50 and piston 55 are inserted into latch cover 54.

Latch cover 54 is fixed to latch 53. Therefore, latch cover 54 moves in the front-rear direction together with latch 53.

In a case of the locking operation of drug injection device 2, latch cover 54 moves forward together with latch 53 to cover device-side engagers 302 in holder 30 (see FIGS. 8 and 11B) of casing 3 from the outside.

In other words, latch cover 54 covers the engagement between device-side engagers 302 and locking engagers 103 of drug cartridge 1 from the outside in a state where the locking operation of drug injection device 2 is completed. Then, latch cover 54 maintains the engagement state between device-side engagers 302 and locking engagers 103 of drug cartridge 1.

As described above, latch cover 54 maintains a state in which drug cartridge 1 is locked to drug injection device 2 (that is, the locked state). A position where latch cover 54 covers device-side engagers 302 from the outside (see FIG. 10B) corresponds to an example of the first position.

Latch cover 54 has detection target 541 (see FIG. 8) on the outer peripheral surface. Detection target 541 moves in the front-rear direction together with latch cover 54. Detection target 541 is a portion detected by first detector 207 (see FIG. 2) and second detector 208 described below.

### (Piston)

Piston 55 has a shaft shape as shown in FIG. 9. Piston 55 has detection target 550 (see FIGS. 10A and 11C) on the outer peripheral surface. Detection target 550 moves in the front-rear direction together with piston 55.

Detection target 550 is a portion detected by basic position detector 209 (see FIGS. 2 and 11C) described below. Specifically, detection target 550 may be a reflective seal. Detection target 550 (specifically, the reflective seal) has an annular shape and is provided over the entire circumference of a part of the outer peripheral surface of piston 55.

When detection target 550 (specifically, the reflective seal) has an annular shape, in the manufacturing process of drug injection device 2, detection target 550 can face basic position detector 209 regardless of the assembly direction of piston 55. Therefore, the work efficiency can be improved.

The reflective seal may not have an annular shape. That is, the reflective seal may be provided at a position facing basic position detector 209 on the outer peripheral surface of piston 55.

Piston 55 is connected to the front end portion of feed screw 50. Specifically, the front end portion of feed screw 50 is inserted into the rear end portion of piston 55.

The thread portion provided at the rear end portion of piston 55 and the thread portion of feed screw 50 are screwed to each other. The thread portion of piston 55 is a female thread portion provided in the inner peripheral surface of the rear end portion of piston 55. The thread portion of feed screw 50 is a male thread portion provided on the outer peripheral surface of feed screw 50.

Piston 55 is inserted into latch 53. The rotation of piston 55 is restricted by latch 53. That is, piston 55 is prevented from rotating. Piston 55 can move in the front-rear direction with respect to latch 53.

In addition, piston 55 moves in the front-rear direction together with feed screw 50. In addition, piston 55 moves in the front-rear direction in response to the rotation of feed screw 50.

When feed screw 50 rotates, piston 55 moves forward based on the screwing between piston 55 and feed screw 50. The operation of piston 55 will be described below.

Feed screw 50 and piston 55 correspond to an example of a second moving member. The second moving member may be regarded as a member that rotates by the motor in the injection operation of drug injection device 2 to push the drug in syringe 13 forward.

### (Other Configurations)

The above is the main configuration of drug injection device 2. In addition to the above-described configurations, drug injection device 2 includes, for example, skin detector 201 (see FIG. 1). Skin detector 201 is provided on the front end portion of drug injection device 2.

Skin detector 201 includes, for example, a capacitive touch sensor. Skin detector 201 detects that the skin (that is, the skin) of the user comes into contact with skin detector 201. Skin detector 201 transmits a detection value to controller 204 described below.

In addition, drug injection device 2 includes plate-shaped main board 203 (see FIG. 2). Various electronic components are mounted on main board 203. In addition, electronic components such as power supply 40 and motor 41 are connected to main board 203.

In the present embodiment, main board 203 is disposed in a state parallel to the XZ plane. Such a configuration can reduce a dimension of drug injection device 2 in the left-right direction (that is, the Y-direction). As a result, drug injection device 2 can be reduced in size.

In addition, controller 204 that integrally controls the operation of drug injection device 2 is mounted on main board 203. The configuration of controller 204 may be a configuration in which an entity such as a CPU, a ROM, and a RAM are connected to one another by a bus or a configuration consisting of a one-chip LSI.

In addition, drug injection device 2 includes RFID antenna 205 (see FIG. 2). RFID antenna 205 transmits the acquired information to controller 204.

Drug injection device 2 includes RFID antenna 205 at each of a position facing flat surface portion 100a of drug cartridge 1 (specifically, case 10) and a position facing flat surface portion 100b of drug cartridge 1 (specifically, case 10) in a state where drug cartridge 1 is attached to drug injection device 2.

In drug injection device 2 of the present embodiment, drug cartridge 1 can be attached to drug injection device 2 in any of a state where flat surface portion 100a is disposed upward and a state where flat surface portion 100b is disposed upward.

Therefore, in any of the state where flat surface portion 100a is disposed upward and the state where flat surface portion 100a is disposed downward, the information (that is, the information related to the drug) stored in RFID tag 101 can be read by RFID antenna 205. Therefore, it is not necessary to provide two RFID tags 101 on drug cartridge 1. Therefore, the cost of drug cartridge 1 can be reduced.

In addition, drug injection device 2 includes cartridge detector 206 (see FIG. 1) in cartridge detection region R1 (see FIG. 10A) at the front end portion of cartridge housing 301.

Cartridge detector 206 detects information indicating that drug cartridge 1 is inserted into cartridge housing 301. Cartridge detector 206 transmits a detection value to controller 204.

In the present embodiment, cartridge detector 206 and controller 204 constitute a cartridge detector configured to detect that drug cartridge 1 is accommodated in cartridge housing 301. It should be noted that cartridge detector 206 alone may detect that drug cartridge 1 is accommodated in cartridge housing 301.

In addition, drug injection device 2 includes first detector 207 (see FIGS. 2 and 10A) and second detector 208 around latch cover 54. First detector 207 is provided rearward of second detector 208. First detector 207 and second detector 208 are provided side by side in the front-rear direction.

First detector 207 and second detector 208 transmit the detection value to controller 204. Controller 204 detects the state of drug injection device 2 based on the detection values of first detector 207 and second detector 208.

Controller 204 detects that drug cartridge 1 is locked to drug injection device 2 based on the detection values of first detector 207 and second detector 208.

In addition, controller 204 detects that drug injection device 2 has ended the puncturing operation based on the detection values of first detector 207 and second detector 208.

In addition, controller 204 detects that drug injection device 2 has ended the needle withdrawal operation based on the detection values of first detector 207 and second detector 208.

Specifically, first detector 207 and second detector 208 are sensors such as a photo interrupter. The detection values of first detector 207 and second detector 208 are different depending on the position of latch cover 54 (specifically, detection target 541).

When detection target 541 of latch cover 54 is detected, first detector 207 and second detector 208 transmit an ON signal to controller 204.

When detection target 541 of latch cover 54 is not detected, first detector 207 and second detector 208 transmit an OFF signal to controller 204.

In the state of drug injection device 2 shown in FIGS. 10A and 10E, neither first detector 207 nor second detector 208 faces detection target 541 of latch cover 54. Therefore, both first detector 207 and second detector 208 transmit the OFF signal to controller 204.

In the locked state of drug injection device 2 shown in FIG. 10B, first detector 207 faces detection target 541 of latch cover 54. Therefore, first detector 207 transmits the ON signal to controller 204.

On the other hand, in the locked state of drug injection device 2 shown in FIG. 10B, second detector 208 does not face detection target 541 of latch cover 54. Therefore, second detector 208 transmits the OFF signal to controller 204.

In the puncture completion state of drug injection device 2 shown in FIG. 10C, first detector 207 does not face detection target 541 of latch cover 54. Therefore, first detector 207 transmits the OFF signal to controller 204.

On the other hand, in the puncture completion state of drug injection device 2 shown in FIG. 10C, second detector 208 faces detection target 541 of latch cover 54. Therefore, second detector 208 transmits the ON signal to controller 204.

In addition, drug injection device 2 includes basic position detector 209 (see FIG. 2) in basic position detection region R2 (see FIGS. 10A and 11C) of cartridge housing 301.

FIG. 11C is an enlarged sectional view of a portion of drug injection device 2 corresponding to basic position detection region R2 in the basic state. The phase around the X axis (in other words, the position around the X axis) of drug injection device 2 in FIG. 11C in the cross section is different from the phase around the X axis (in other words, the position around the X axis) of drug injection device 2 in FIG. 10A in the cross section.

Basic position detector 209 is a non-contact and optical sensor (specifically, a photoreflector).

Basic position detector 209 is directly or indirectly supported by inner case element 31. As shown in FIG. 11C, basic position detector 209 is provided at a position facing detection target 550 of piston 55 with a gap in a state where piston 55 is positioned at the basic position (in other words, the basic state of drug injection device 2).

In this state, through-hole 531 of latch 53 is present between basic position detector 209 and detection target 550. Therefore, in the state shown in FIG. 11C, basic position detector 209 and detection target 550 face each other in the radial direction of latch 53 via through-hole 531.

It should be noted that, in a state other than the basic state of drug injection device 2, detection target 550 is covered by latch 53 as a result of the relative movement between latch 53 and piston 55. Basic position detector 209, detection target 550, and latch 53 are in a positional relationship in which basic position detector 209 can detect detection target 550 only in the basic state (the state shown in FIGS. 10A, 10F, and 11C) of drug injection device 2.

FIGS. 10A to 10F schematically show the positional relationship between basic position detector 209, detection target 550, and through-hole 531 in each state of drug injection device 2.

Drug injection device 2 shown in FIG. 10A is in the basic state. Drug injection device 2 shown in FIG. 10B is in the locked state. Drug injection device 2 shown in FIG. 10C is in the puncture completion state. Drug injection device 2 shown in FIG. 10D is in the injection completion state. Drug injection device 2 shown in FIG. 10E is in the needle withdrawal completion state. Drug injection device 2 shown in FIG. 10F is in the basic state. Details of each state of drug injection device 2 shown in FIGS. 10A to 10F will be described below.

As shown in FIGS. 10A and 10F, in the basic state of drug injection device 2, basic position detector 209 and detection target 550 face each other in the radial direction of piston 55 via through-hole 531. That is, basic position detector 209 can detect detection target 550.

In addition, as shown in FIG. 10B, in the locked state of drug injection device 2, latch 53 and piston 55 move forward (in other words, in the puncturing direction) further than in the basic state. In this state, basic position detector 209 and detection target 550 do not face each other in the radial direction of piston 55. Therefore, basic position detector 209 cannot detect detection target 550.

In addition, as shown in FIG. 10C, in the puncture completion state of drug injection device 2, latch 53 and piston 55 move forward (in other words, in the puncturing direction) further than in the locked state. In this state, basic position detector 209 and detection target 550 do not face each other in the radial direction of piston 55. Therefore, basic position detector 209 cannot detect detection target 550. It should be noted that, in this state, basic position detector 209 is covered from the outer side in the radial direction by slider 51.

In addition, as shown in FIG. 10D, in the injection completion state of drug injection device 2, piston 55 moves forward (in other words, in the puncturing direction) further than in the puncture completion state. In this state, basic position detector 209 and detection target 550 do not face each other in the radial direction of piston 55. Therefore, basic position detector 209 cannot detect detection target 550. It should be noted that, in this state, basic position detector 209 is covered from the outer side in the radial direction by slider 51.

In addition, as shown in FIG. 10E, in the needle withdrawal completion state of drug injection device 2, latch 53 moves rearward (in other words, in the needle withdrawal direction) further than in the injection completion state. In this state, basic position detector 209 and detection target 550 do not face each other in the radial direction of piston 55. Therefore, basic position detector 209 cannot detect detection target 550. Details of the operation of drug injection device 2 will be described below.

As described above, basic position detector 209 detects that piston 55 is positioned at the basic position (specifically, the position shown in FIGS. 10A, 10F, and 11C).

In other words, basic position detector 209 detects that drug injection device 2 is in the basic state (FIGS. 10A, 10F, and 11C). Furthermore, in other words, basic position detector 209 detects that the piston is completely returned after drug injection device 2 ends the needle withdrawal operation. Basic position detector 209 detects that all of a series of injection operations of drug injection device 2 are ended based on the position of piston 55.

Specifically, in a state where piston 55 is positioned at the basic position, detection target 550 of piston 55 (see FIGS. 10A and 11C) faces basic position detector 209 via through-hole 531 of latch 53 (see FIG. 11C). As described above, basic position detector 209 is provided at the position facing detection target 550 of piston 55 in a state where piston 55 is positioned at the basic position.

In a state where piston 55 is positioned at the basic position, basic position detector 209 detects detection target 550 of piston 55. Basic position detector 209 transmits the detection value to controller 204.

On the other hand, in a state where piston 55 is not positioned at the basic position (for example, the state shown in FIG. 10C), detection target 550 of piston 55 does not face basic position detector 209. Therefore, basic position detector 209 does not detect detection target 550 of piston 55.

In addition, drug injection device 2 includes a notification means that notifies the user of information. The notification means is configured by, for example, LED light 211 provided on an upper surface of casing 3. Specifically, drug injection device 2 may change a display mode of LED light 211 according to the type of the drug with which drug cartridge 1 is filled.

For example, drug injection device 2 may change the color of LED light 211 according to the type of the drug with which drug cartridge 1 is filled. The user can recognize that the drug to be administered is correct by checking the color of LED light 211. This configuration contributes to improving safety in a case of self-injection.

In addition, drug injection device 2 may change the display mode of LED light 211 according to the state of drug injection device 2 (in other words, according to the progress) in a case of self-injection.

For example, drug injection device 2 turns off LED light 211 in a state before drug injection device 2 is used. Next, drug injection device 2 turns on LED light 211 in a state where drug cartridge 1 is attached to drug injection device 2 (in other words, the locked state of drug injection device 2).

In addition, drug injection device 2 turns on LED light 211 in a state where drug injection device 2 is in the process of preparation for administration (in other words, the preparation state of drug injection device 2), as in the locked state of drug injection device 2.

In addition, drug injection device 2 causes LED light 211 to slowly blink in a state where drug injection device 2 is in a state in which the drug can be administered (in other words, the state in which drug injection device 2 is available for administration).

In addition, in a state where the drug is being administered by drug injection device 2 (in other words, the administration state of drug injection device 2), drug injection device 2 causes LED light 211 to slowly blink in a color different from that used in the state in which the drug can be administered by drug injection device 2. It should be noted that, in the administration state, drug injection device 2 performs the puncturing operation and the injection operation of drug injection device 2 described below.

In addition, in a state where the administration of the drug by drug injection device 2 is completed (in other words, the administration completion state of drug injection device 2), drug injection device 2 causes LED light 211 to blink slightly faster (for example, at 2-second intervals) in a color the same as in the administration state of drug injection device 2.

In addition, in a state where drug injection device 2 is performing the needle withdrawal operation (in other words, an origin return state of drug injection device 2), drug injection device 2 causes LED light 211 to blink in a color different from that used in the state in which the drug can be administered by drug injection device 2 and the administration state of drug injection device 2.

As described above, the user can recognize the progress of the self-injection by visually recognizing LED light 211 in a case of self-injection.

In addition, when there is an error in the operation of the user or the operation of drug injection device 2 in a case of self-injection, drug injection device 2 may change the display mode of LED light 211 according to the type of the error.

Specifically, when injection button 202 is pressed in a state where the skin of the user does not come into contact with skin detector 201, drug injection device 2 causes LED light 211 to display in a display mode indicating that the operation of the user is in error. The user can recognize that there is an error in the operation by looking at LED light 211.

In addition, when injection button 202 is not pressed in a state where drug injection device 2 is in the state in which the drug can be administered (in other words, the state in which drug injection device 2 is available for administration), drug injection device 2 may change the display mode of LED light 211 to prompt the user to press injection button 202. In this case, drug injection device 2 may cause LED light 211 to slowly blink in an orange color.

In addition, drug injection device 2 may change the display mode of LED light 211 according to the charging status of power supply 40.

In addition, drug injection device 2 may change the display mode of LED light 211 according to a communication connection state between drug injection device 2 and a mobile terminal of the user. For example, when the user connects drug injection device 2 to the mobile terminal by Bluetooth (registered trademark), the display mode of LED light 211 may be changed according to a pairing status between drug injection device 2 and the mobile terminal.

It should be noted that the display mode of LED light 211 is not limited to the above-described case. In addition, the notification means is not limited to the LED light. The notification means may be a notification means that outputs a voice or a notification means that displays a character on a display.

### (Operation of Drug Injection Device)

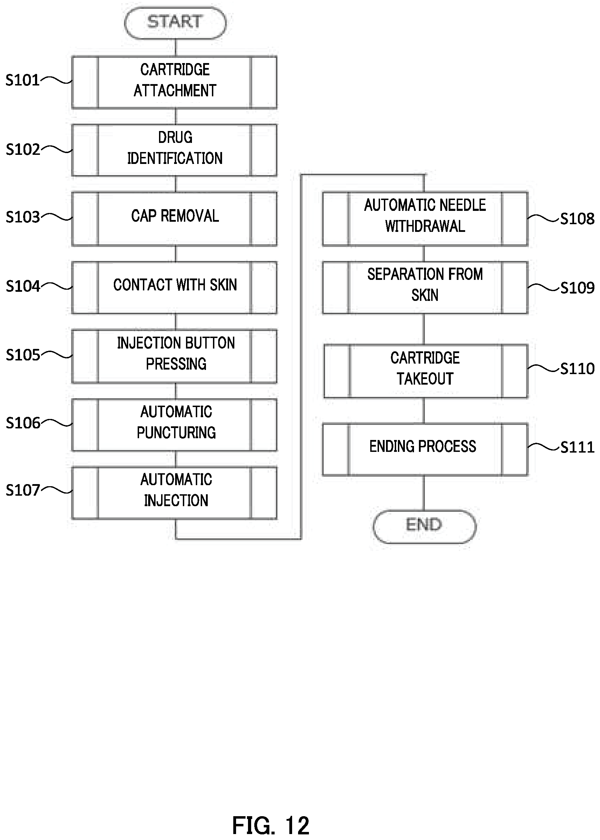

Next, the operation of drug injection device 2 in a case of self-injection will be described. FIG. 12 is a flowchart showing a process performed when the user performs the self-injection. First, the process shown in FIG. 12 will be described. Then, details of each process shown in FIG. 12 will be described. The subject of the process shown in FIG. 12 is the user or drug injection device 2.

In step S101 of FIG. 12, the user performs a cartridge attachment process. The cartridge attachment process is a process of attaching drug cartridge 1 to drug injection device 2.

Next, in step S102 of FIG. 12, drug injection device 2 performs a drug identification process. Drug injection device 2 identifies the drug with which syringe 13 of drug cartridge 1 is filled, by the drug identification process.

Next, in step S103 of FIG. 12, the user performs a cap removal process. The user removes cap 11 from drug cartridge 1 attached to drug injection device 2 by the cap removal process. In this case, needle shield 133 of drug cartridge 1 is removed from drug cartridge 1 together with cap 11.

Next, in step S104 of FIG. 12, the user performs a process (hereinafter, referred to as a "skin contact process") of bringing skin detector 201 of drug injection device 2 into contact with the skin of the user.

Next, in step S105 of FIG. 12, the user performs an injection button pressing process. In the injection button pressing process, the user presses injection button 202 (see FIG. 1) of drug injection device 2.

Next, in step S106 of FIG. 12, drug injection device 2 performs an automatic puncturing process. In the automatic puncturing process, drug injection device 2 punctures the skin of the user with needle 131 of syringe 13.

Next, in step S107 of FIG. 12, drug injection device 2 performs an automatic injection process. In the automatic puncturing process, drug injection device 2 injects into the user the drug with which syringe 13 is filled.

Next, in step S108 of FIG. 12, drug injection device 2 performs an automatic needle withdrawal process. In the automatic needle withdrawal process, drug injection device 2 removes needle 131 of syringe 13 from the skin of the user.

Next, in step S109 of FIG. 12, the user performs a process of separating skin detector 201 of drug injection device 2 from the skin of the user.

Next, in step S110 of FIG. 12, the user performs a cartridge removal process. In the cartridge removal process, the user takes out drug cartridge 1 from drug injection device 2.

Finally, in step S111 of FIG. 12, the user ends the self-injection.

### (Cartridge Attachment Process)

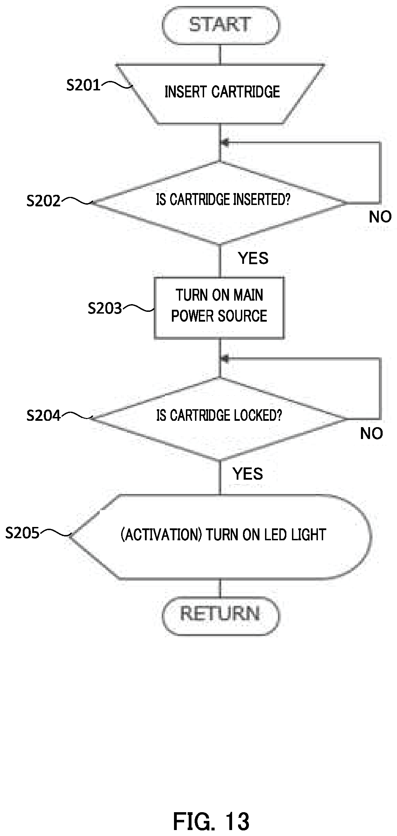

Next, details of each process shown in FIG. 12 will be described. First, the cartridge attachment process of step S101 of FIG. 12 will be described with reference to FIG. 13.

In step S201 of FIG. 13, the user inserts drug cartridge 1 into cartridge housing 301 (see FIG. 1) of drug injection device 2.

FIG. 10A is a sectional view showing a state in which drug cartridge 1 is inserted into drug injection device 2. In the state shown in FIG. 10A, drug injection device 2 has not locked drug cartridge 1. That is, drug injection device 2 has not performed the locking operation. Drug injection device 2 shown in FIG. 10A is in the basic state.

Next, in step S202 of FIG. 13, controller 204 of drug injection device 2 determines whether or not drug cartridge 1 is inserted into drug injection device 2. Controller 204 determines whether or not drug cartridge 1 is inserted into drug injection device 2 based on the detection value of cartridge detector 206 (see FIG. 2).

When the insertion of drug cartridge 1 is not detected by cartridge detector 206 (in step S202 of FIG. 13, "NO"), controller 204 repeats the process of step S202 of FIG. 13.

When the insertion of drug cartridge 1 is detected by cartridge detector 206 (in step S202 of FIG. 13, "YES"), controller 204 advances the control processing to step S203 of FIG. 13.

Next, in step S203 of FIG. 13, controller 204 turns on the main power source of drug injection device 2.

Next, in step S204 of FIG. 13, controller 204 determines whether or not drug cartridge 1 is locked to drug injection device 2 based on the detection values of first detector 207 and second detector 208. In other words, in step S204 of FIG. 13, controller 204 determines whether or not drug injection device 2 is in the locked state.

When drug cartridge 1 is not locked to drug injection device 2 (in step S204 of FIG. 13, "NO"), controller 204 repeats the process of step S204 of FIG. 13.

When drug cartridge 1 is locked to drug injection device 2 (in step S204 of FIG. 13, "YES"), controller 204 advances the control processing to step S205 of FIG. 13.

As described above, in step S204 of FIG. 13, controller 204 determines whether or not the locking operation of drug injection device 2 is ended.

Here, the locking operation of drug injection device 2 will be described. FIG. 10B is a sectional view showing a state of drug injection device 2 after the locking operation is ended. When the locking operation is performed, drug injection device 2 transitions from the basic state shown in FIG. 10A to the locked state shown in FIG. 10B.

In the locking operation, drug injection device 2 drives motor 41. Motor 41 is driven in the forward direction. Then, motor 41 is driven by a predetermined amount in the forward direction. The rotation amount of motor 41 in the locking operation may be, for example, a rotation amount corresponding to 1/4 rotation (that is, 90°) of feed screw 50.

A direction in which each element of actuator 5 rotates in response to the rotation of motor 41 in the forward direction is referred to as a first rotational direction. On the other hand, a direction in which each element of actuator 5 rotates in response to the rotation of motor 41 in the reverse direction is referred to as a second rotational direction.

When motor 41 rotates, the rotation of motor 41 is transmitted to actuator 5 (specifically, feed screw 50) via decelerator 42 and transmitter 43. Then, feed screw 50 rotates by a predetermined amount (for example, 1/4 rotation) in a predetermined direction. The rotational direction of feed screw 50 at this time is the first rotational direction.

When feed screw 50 rotates, torque limiter 52 rotates together with feed screw 50. In this case, since the rotation of slider 51 in the first rotational direction is not restricted, the torque acting on torque limiter 52 is equal to or less than a predetermined value. Therefore, the rotation of feed screw 50 is transmitted to slider 51 via torque limiter 52.

When slider 51 rotates in the first rotational direction, guide groove 510 of slider 51 is guided by guide protrusion 310 (see FIG. 9) of casing 3 (specifically, inner case element 31), and slider 51 moves forward by a predetermined amount.

In this case, slider 51 moves forward by a predetermined amount from the rear end of the stroke of slider 51. The forward movement amount of slider 51 corresponds to the rotation amount of motor 41. A position of slider 51 after the movement is also referred to as the locked position of slider 51.

When slider 51 moves forward, latch 53 and latch cover 54 move forward by a predetermined amount together with slider 51. Then, latch cover 54 moves from the position shown in FIG. 10A (also referred to as the basic position) to the position shown in FIG. 10B (also referred to as the locked position).

In the locked position shown in FIG. 10B, latch cover 54 covers device-side engagers 302 in holder 30 of casing 3 from the outside (see FIG. 11B). Then, latch cover 54 maintains the engagement state between device-side engagers 302 and locking engagers 103 of drug cartridge 1. That is, latch cover 54 locks drug cartridge 1 to drug injection device 2. Then, drug injection device 2 is in the locked state.

Here, the states of device-side engagers 302, locking engagers 103, and latch cover 54 in the states of drug injection device 2 shown in FIGS. 10A and 10B will be described with reference to FIGS. 11A and 11B.

FIG. 11A is an enlarged sectional view of a portion corresponding to the X1 portion of FIG. 10A, which is taken at a position shifted by a predetermined angle in the circumferential direction. In addition, FIG. 11B is an enlarged sectional view of a portion corresponding to the X2 portion of FIG. 10B, which is taken at a position shifted by a predetermined angle in the circumferential direction. FIGS. 11A and 11B are sectional views showing the states of device-side engagers 302, locking engagers 103, and latch cover 54.

As shown in FIG. 10A, in a state where drug cartridge 1 is inserted into drug injection device 2 and drug cartridge 1 is not locked to drug injection device 2, claw portions 302a of device-side engagers 302 are inserted into locking engagers 103 from the outer side in the radial direction of drug cartridge 1 (specifically, case 10) as shown in FIG. 11A.

As shown in FIG. 11A, claw portions 302a have a trapezoidal sectional shape. In addition, claw portions 302a have an inclined surface on a rear end surface and a front end surface.

When case 10 of drug cartridge 1, from the front side, enters the inside of device-side engagers 302, device-side engagers 302 are elastically deformed to be expanded in diameter. In the present embodiment, since the front end surfaces of claw portions 302a are the inclined surface, case 10 easily enters the inside of device-side engagers 302.

Then, when locking engagers 103 of case 10 proceed to positions corresponding to claw portions 302a of device-side engagers 302, device-side engagers 302 are elastically deformed to be reduced in diameter. As a result, claw portions 302a are engaged with locking engagers 103 from the outer side in the radial direction. In a state where claw portions 302a and locking engagers 103 are engaged with each other in this way, drug cartridge 1 is temporarily fixed to drug injection device 2.

Thereafter, as described above, when latch cover 54 moves forward, latch cover 54 covers device-side engagers 302 from the outside as shown in FIG. 11B. As a result, drug injection device 2 is in the locked state. That is, drug cartridge 1 is fixed to drug injection device 2.

As shown in FIG. 10B, in the locked state of drug injection device 2, detection target 541 of latch cover 54 faces first detector 207. Therefore, first detector 207 detects detection target 541 of latch cover 54. In this state, first detector 207 transmits the ON signal to controller 204.

On the other hand, in the locked state of drug injection device 2, detection target 541 of latch cover 54 does not face second detector 208. Therefore, second detector 208 does not detect detection target 541 of latch cover 54. In this state, second detector 208 transmits the OFF signal to controller 204.

Then, controller 204 detects that drug injection device 2 is in the locked state based on the detection values acquired from first detector 207 and second detector 208.

As described above, the locking operation of drug injection device 2 is performed by the actuation of feed screw 50, slider 51, torque limiter 52, latch 53, and latch cover 54 of actuator 5. Feed screw 50, slider 51, torque limiter 52, latch 53, and latch cover 54 correspond to an example of a locking operation mechanism.

Hereinafter, the description will again be given with reference to FIG. 13. In step S205 of FIG. 13, controller 204 turns on LED light 211 in a display mode indicating that drug injection device 2 is activated. Then, controller 204 ends the cartridge attachment process.

As described above, when the cartridge attachment process is ended, drug injection device 2 is in the locked state shown in FIG. 10B.

### (Drug Identification Process)

Next, the drug identification process of step S102 of FIG. 12 will be described. In the drug identification process, RFID antenna 205 (see FIG. 2) of drug injection device 2 reads information related to the drug from RFID tag 101 (see FIG. 1) disposed in drug cartridge 1. Then, RFID antenna 205 transmits the acquired information related to the drug to controller 204.

The information related to the drug includes a unique ID, information related to the type of the drug, information related to the lot, and information related to the expiration date of the drug. Controller 204 may transmit the information related to the drug to a mobile terminal that is wirelessly connected to drug injection device 2.

It should be noted that a wireless communication method for connecting drug injection device 2 and the mobile terminal may be various communication methods such as Wireless Fidelity (Wi-Fi) (registered trademark) or Bluetooth (registered trademark).

The mobile terminal may notify the user of the information related to the drug acquired from drug injection device 2. The notification means (for example, LED light 211) is as described above.

It should be noted that the information related to the drug may include information indicating that drug cartridge 1 has not yet been used and/or information indicating that drug cartridge 1 has already been used. When drug cartridge 1 is used, controller 204 may notify the user of this fact.

In addition, in the drug identification process, when the drug with which drug cartridge 1 is filled is a drug that does not match drug injection device 2, controller 204 may notify the user of this fact. In addition, when the drug with which drug cartridge 1 is filled is a drug that does not match drug injection device 2, controller 204 may release the locking of drug cartridge 1 and discharge drug cartridge 1 from drug injection device 2.

### (Skin Contact Process)

Next, the skin contact process of step S104 of FIG. 12 will be described. In the skin contact process, drug injection device 2 may notify the user of a message prompting the user to bring skin detector 201 of drug injection device 2 into contact with the skin of the user. Such a message may be notified by drug injection device 2 or may be notified by the mobile terminal wirelessly connected to drug injection device 2.

The user who receives the message as described above brings skin detector 201 of drug injection device 2 into contact with the skin of the user.

When skin detector 201 detects that the skin of the user comes into contact with skin detector 201, skin detector 201 transmits the detection value to controller 204. When the detection value is acquired from skin detector 201, controller 204 switches the state of injection button 202 of drug injection device 2 to a state in which the operation input can be received.

In other words, injection button 202 is in a state in which the operation input can be received only in a state where the skin of the user is in contact with skin detector 201. Such a configuration contributes to suppressing the malfunction of drug injection device 2.

It should be noted that, in the case of drug injection device 2 shown in FIG. 1, drug injection device 2 includes skin detector 201 on one side half portion (specifically, the half portion on the positive Y-direction) of the distal end surface. On the other hand, drug injection device 2 includes cartridge housing 301 on the other side half portion (specifically, the half portion on the negative Y-direction) of the distal end surface.

Therefore, in the skin contact process, only skin detector 201 comes into contact with the skin of the user. Skin detector 201 corresponds to an example of a first skin contactor. That is, in the case of drug injection device 2 shown in FIG. 1, only the first skin contactor provided on one side half portion of the distal end portion of drug injection device 2 comes into contact with the skin of the user.

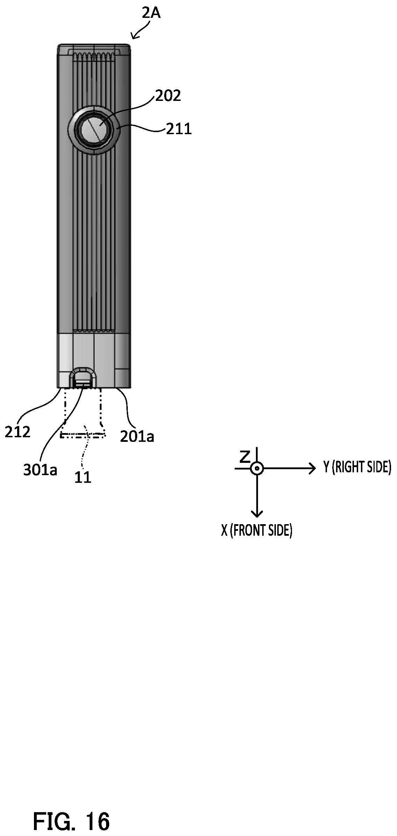

FIG. 16 is a diagram showing a variation of the drug injection device. Drug injection device 2A shown in FIG. 16 includes skin detector 201a (in other words, a first skin contactor) on one side half portion (specifically, the half portion on the positive Y-direction) of the distal end surface.

In addition, drug injection device 2A includes second skin contactor 212 on the other side half portion (specifically, the half portion on the negative Y-direction) of the distal end surface. In addition, drug injection device 2A includes cartridge housing 301a between skin detector 201a and second skin contactor 212 in the Y-direction.

Other configurations of drug injection device 2A are the same as the configurations of drug injection device 2 described above. Therefore, second skin contactor 212 of drug injection device 2A may be provided in drug injection device 2. In addition, the description of drug injection device 2A may be appropriately replaced with the description of drug injection device 2 described above.

Skin detector 201a (in other words, the first skin contactor) and second skin contactor 212 of drug injection device 2A are positioned on the same plane parallel to the YZ-direction. Therefore, in the case of drug injection device 2A, in the skin contact process, skin detector 201a (in other words, first skin contactor) and second skin contactor 212 come into contact with the skin of the user.

Therefore, an area of drug injection device 2A that comes into contact with the skin of the user is increased. As a result, the contact state between drug injection device 2A and the skin of the user is stabilized. In addition, the configurations of skin detector 201a (in other words, the first skin contactor) and second skin contactor 212 can make the user aware that drug injection device 2A is to make contact with the skin in a planar manner without tilting drug injection device 2A.

### (Injection Button Pressing Process)

Next, the injection button pressing process of step S105 of FIG. 12 will be described. In the injection button pressing process, drug injection device 2 may notify the user of a message prompting the user to press injection button 202 (see FIG. 1) of drug injection device 2. Such a message may be notified by drug injection device 2 or may be notified by the mobile terminal wirelessly connected to drug injection device 2.

The user who receives the message as described above presses injection button 202 of drug injection device 2. When the pressing of injection button 202 of drug injection device 2 is detected, controller 204 ends the injection button pressing process. When the injection button pressing process is ended, drug injection device 2 is in a state in which the automatic puncturing process, the automatic injection process, and the automatic needle withdrawal process described below can be performed.

### (Automatic Puncturing Process/Automatic Injection Process)

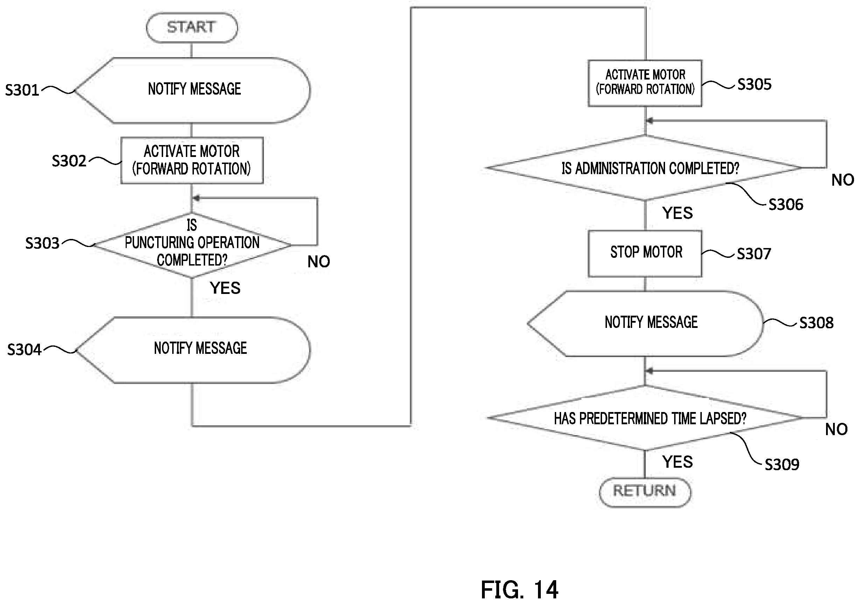

Next, the automatic puncturing process of step S106 and the automatic injection process of step S107 of FIG. 12 will be described with reference to FIG. 14.

In the flowchart shown in FIG. 14, drug injection device 2 mainly performs the automatic puncturing process and the automatic injection process. The automatic puncturing process is performed before the automatic injection process. When the automatic puncturing process is ended, drug injection device 2 automatically performs the automatic injection process.

First, the automatic puncturing process will be described. The operation of drug injection device 2 in the automatic puncturing process is the puncturing operation. When the injection button pressing process of step S105 of FIG. 12 is ended, drug injection device 2 automatically starts the puncturing operation.

In step S301 of FIG. 14, drug injection device 2 notifies the user of a message indicating that the puncturing is performed. Such a message may be notified by drug injection device 2 or may be notified by the mobile terminal wirelessly connected to drug injection device 2.

In step S302 of FIG. 14, drug injection device 2 drives motor 41. Motor 41 is driven in the forward direction. When motor 41 is driven, the power (that is, the rotation) of motor 41 is transmitted to actuator 5 via decelerator 42 and transmitter 43 via driver 4. Then, actuator 5 performs the puncturing operation.

A direction in which each element of actuator 5 rotates in response to the rotation of motor 41 in the forward direction is referred to as a first rotational direction. On the other hand, a direction in which each element of actuator 5 rotates in response to the rotation of motor 41 in the reverse direction is referred to as a second rotational direction.

Here, the puncturing operation of drug injection device 2 will be described. FIG. 10B is a sectional view showing a state (that is, the locked state) of drug injection device 2 before the puncturing operation is performed. Then, FIG. 10C is a sectional view showing a state (that is, the puncture completion state) of drug injection device 2 after the puncturing operation is performed.

In this way, when the puncturing operation is performed, drug injection device 2 transitions from the locked state shown in FIG. 10B to the puncture completion state shown in FIG. 10C.

In a case of the puncturing operation, drug injection device 2 drives motor 41 in the forward direction. When motor 41 rotates, the rotation of motor 41 is transmitted to actuator 5 (specifically, feed screw 50) via decelerator 42 and transmitter 43. Then, feed screw 50 rotates in the first rotational direction.

When feed screw 50 rotates, torque limiter 52 rotates in the first rotational direction together with feed screw 50. In this case, since the rotation of slider 51 in the first rotational direction is not restricted, the torque acting on torque limiter 52 is equal to or less than a predetermined value.

Therefore, the rotation of feed screw 50 is transmitted to slider 51 via torque limiter 52. In this state, torque limiter 52 connects feed screw 50 and slider 51.

When slider 51 rotates, guide groove 510 of slider 51 is guided by guide protrusion 310 (see FIG. 9) of casing 3 (specifically, inner case element 31), and slider 51 moves forward by a predetermined amount.

Slider 51 moves forward until guide protrusion 310 is positioned at the end point (rear end) of guide groove 510. In this case, slider 51 moves from the locked position of slider 51 to the front end of the stroke.

At the front end of the stroke, slider 51 is restricted from rotating by guide protrusion 310. In addition, at the front end of the stroke, slider 51 is also restricted from moving forward.

When slider 51 moves forward, latch 53 and latch cover 54 move forward together with slider 51. Then, the front end portion of latch 53 pushes the rear end portion of syringe 13 (specifically, syringe body 130) forward.

As a result, entire syringe 13 moves forward. When entire syringe 13 moves forward by a predetermined amount, needle 131 of syringe 13 protrudes forward from the front end of drug injection device 2 as shown in FIG. 10C. Then, the puncturing operation of drug injection device 2 is ended.

As described above, the puncturing operation of drug injection device 2 is performed by the operation of feed screw 50, slider 51, torque limiter 52, and latch 53 of actuator 5. Feed screw 50, slider 51, torque limiter 52, and latch 53 correspond to an example of a puncturing operation mechanism.

When the puncturing operation of drug injection device 2 is ended, controller 204 performs the injection operation in a state where motor 41 has been driven. In other words, controller 204 continues to drive motor 41 between the puncturing operation and the injection operation. That is, controller 204 continuously performs the puncturing operation and the injection operation. It should be noted that controller 204 may stop motor 41 between the puncturing operation and the injection operation. That is, controller 204 may perform the puncturing operation and the injection operation in a discontinuous manner.

As shown in FIG. 10C, in the puncture completion state of drug injection device 2, detection target 541 of latch cover 54 faces second detector 208. Therefore, second detector 208 detects detection target 541 of latch cover 54. In this state, second detector 208 transmits the ON signal to controller 204.

On the other hand, in the puncture completion state of drug injection device 2, detection target 541 of latch cover 54 does not face first detector 207. Therefore, first detector 207 does not detect detection target 541 of latch cover 54. In this state, first detector 207 transmits the OFF signal to controller 204.

Then, controller 204 detects that drug injection device 2 is in a state of having completed the puncturing operation (that is, the puncture completion state) based on the detection values acquired from first detector 207 and second detector 208.

As described above, in the present embodiment, needle 131 of syringe 13 protrudes forward from the front end of drug injection device 2 only after the puncturing operation of drug injection device 2 is ended. This configuration contributes to improving safety in a case of self-injection.

Hereinafter, the description will again be given with reference to FIG. 14. In step S303 of FIG. 14, controller 204 determines whether or not the puncturing operation is completed. The determination method is as described above.

When it is determined that the puncturing operation is not completed in step S303 of FIG. 14, controller 204 repeats step S303.

On the other hand, when it is determined that the puncturing operation is completed in step S303 of FIG. 14, controller 204 advances the control processing to step S304.

In step S304 of FIG. 14, controller 204 notifies the user of a message indicating the injection. Such a message may be notified by drug injection device 2 or may be notified by the mobile terminal wirelessly connected to drug injection device 2. When the process of step S304 of FIG. 14 is ended, drug injection device 2 performs the automatic injection process.

In step S305 of FIG. 14, drug injection device 2 drives motor 41. In this case, motor 41 is driven in the forward direction. When motor 41 is driven, the power (that is, the rotation) of motor 41 is transmitted to actuator 5 via driver 4. Then, actuator 5 performs the injection operation.

Here, the injection operation of drug injection device 2 will be described. FIG. 10C is a sectional view showing a state (that is, the puncture completion state) of drug injection device 2 before the injection operation is performed. Then, FIG. 10D is a sectional view showing a state (that is, the injection completion state) of drug injection device 2 after the injection operation is performed.

In this way, when the injection operation is performed, drug injection device 2 transitions from the puncture completion state shown in FIG. 10C to the injection completion state shown in FIG. 10D.

In a case of the injection operation, drug injection device 2 drives motor 41 in the forward direction. When motor 41 rotates, the rotation of motor 41 is transmitted to actuator 5 (specifically, feed screw 50) via decelerator 42 and transmitter 43. Then, feed screw 50 rotates in the first rotational direction.

When feed screw 50 rotates, the rotation of feed screw 50 is transmitted to torque limiter 52. In the puncture completion state of drug injection device 2 shown in FIG. 10C, slider 51 has moved to the rear end of the stroke.

In other words, guide protrusion 310 of casing 3 (specifically, inner case element 31) is positioned at the end point of guide groove 510 of slider 51. In this state, slider 51 is restricted from rotating in the first rotational direction by guide protrusion 310.

Therefore, the torque acting on torque limiter 52 from feed screw 50 is larger than the predetermined value.

When the torque acting on torque limiter 52 is larger than the predetermined value, torque limiter 52 is in a state (that is, the non-transmission state of the torque limiter) in which the rotation of feed screw 50 is not transmitted to slider 51. That is, torque limiter 52 disconnects feed screw 50 from slider 51.

As described above, in the puncture completion state of drug injection device 2, slider 51 does not rotate. Therefore, slider 51 does not move forward. Therefore, feed screw 50 rotates without moving forward.

Piston 55 is connected to the front end portion of feed screw 50. Then, piston 55 is screwed to feed screw 50 so as to be prevented from rotating. Therefore, when feed screw 50 rotates, piston 55 moves forward based on the rotation of feed screw 50.

The front end of piston 55 comes into contact with syringe-side piston 132 of syringe 13 in the puncture completion state of drug injection device 2. Therefore, when piston 55 moves forward, syringe-side piston 132 is pushed forward.

When syringe-side piston 132 moves forward, the drug with which syringe 13 is filled is injected from the distal end of needle 131. Then, the injection operation of drug injection device 2 is ended.

As described above, the injection operation of drug injection device 2 is performed by the operation of feed screw 50 and piston 55 of actuator 5. Feed screw 50 and piston 55 correspond to an example of an injection operation mechanism.

Hereinafter, the description will be given again with reference to FIG. 14. In step S306 of FIG. 14, controller 204 determines whether or not the administration of the drug is completed.

Specifically, controller 204 determines whether or not piston 55 (in other words, syringe-side piston 132) has moved forward by a distance at which the drug in a preset amount can be injected. That is, controller 204 determines whether or not to end the injection operation of drug injection device 2.

It should be noted that the administration amount is set in advance and stored in memory 210 (see FIG. 2) of drug injection device 2. Memory 210 may store the administration amount in association with the type of the drug and the identification information of the user.

In step S306 of FIG. 14, controller 204 acquires a forward movement amount of piston 55 (in other words, the position in the front-rear direction) based on the detection value of position detector 200 disposed on motor 41. Then, controller 204 determines whether or not the administration of the drug is completed based on the acquired forward movement amount of piston 55.

When it is determined that the administration of the drug is not completed in step S306 of FIG. 14, controller 204 continues the injection operation of drug injection device 2 and repeats step S306. In this way, the process of step S306 is a process that is always performed in a case of the injection operation of drug injection device 2.

When it is determined that the administration of the drug is completed in step S306 of FIG. 14, controller 204 advances the control processing to step S307.

In step S307 of FIG. 14, controller 204 stops motor 41. Then, the injection operation of drug injection device 2 is ended.

In step S308 of FIG. 14, controller 204 notifies the user of a message indicating that the state where skin detector 201 of drug injection device 2 is in contact with the skin is maintained. Such a message may be notified by drug injection device 2 or may be notified by the mobile terminal wirelessly connected to drug injection device 2.

In step S309 of FIG. 14, controller 204 determines whether or not a predetermined time has elapsed from the end of the process of step S308. In other words, controller 204 determines whether or not a time during which the user maintains the state where skin detector 201 of drug injection device 2 is in contact with the skin has elapsed for a predetermined time.

When it is determined that the predetermined time has not elapsed in step S309 of FIG. 14, controller 204 repeats step S309.

When it is determined that the predetermined time has elapsed in step S309 of FIG. 14, controller 204 ends the control processing.

### (Automatic Needle Withdrawal Process)

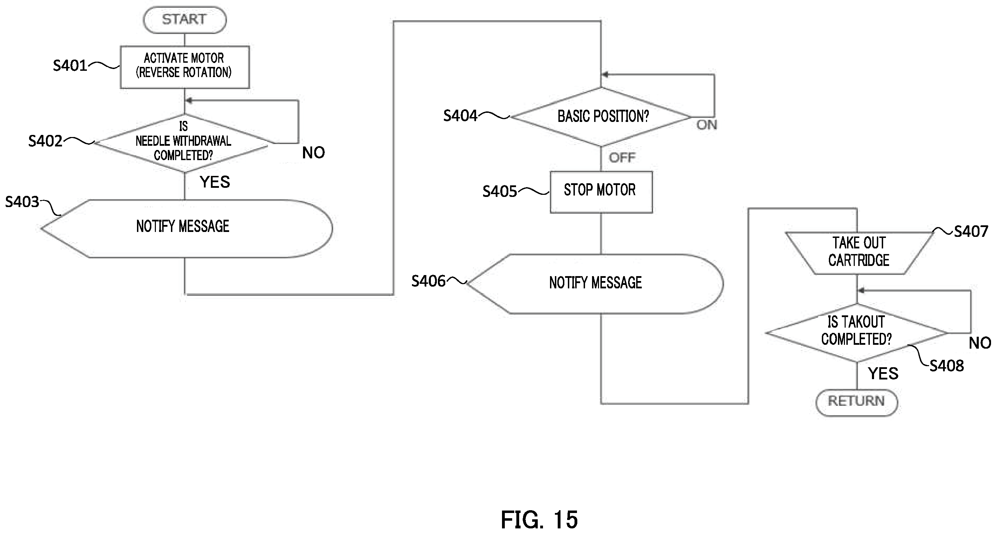

Next, the processes of from the automatic needle withdrawal process of step S108 to the process of step S110 of FIG. 12 will be described with reference to FIG. 15.

In the flowchart shown in FIG. 15, drug injection device 2 mainly performs the automatic needle withdrawal process and the unlocking process. The automatic needle withdrawal process is performed after the automatic injection process described above. In addition, drug injection device 2 performs the unlocking process in the course of the automatic needle withdrawal process.

In step S401 of FIG. 15, drug injection device 2 starts the automatic needle withdrawal process. The operation of drug injection device 2 in the automatic needle withdrawal process is the needle withdrawal operation. Specifically, in step S401 of FIG. 15, drug injection device 2 drives motor 41. In this case, motor 41 is driven in the reverse direction.

The rotational direction of motor 41 in the needle withdrawal operation of drug injection device 2 is a direction opposite to the rotational direction of motor 41 in the puncturing operation and the injection operation of drug injection device 2.

A direction in which each element of actuator 5 rotates in response to the rotation of motor 41 in the reverse direction is the second rotational direction.

When motor 41 is driven, actuator 5 performs the needle withdrawal operation based on the power (that is, the rotation) of motor 41.

FIG. 10D is a sectional view showing a state (that is, the injection completion state) of drug injection device 2 before the needle withdrawal operation is performed. Then, FIG. 10E is a sectional view showing a state (also referred to as a needle withdrawal completion state) of drug injection device 2 after the needle withdrawal is completed. In addition, FIG. 10F is a sectional view showing a state of drug injection device 2 after the entire needle withdrawal operation is completed.

In this way, when the needle withdrawal operation is performed, drug injection device 2 transitions from the injection completion state shown in FIG. 10D to the basic state shown in FIG. 10F through the state shown in FIG. 10E. In the state shown in FIG. 10E, needle 131 is withdrawn from the skin of the user. That is, in the state shown in FIG. 10E, the needle withdrawal is completed. After needle 131 is withdrawn from the skin of the user, drug injection device 2 transitions from the state shown in FIG. 10E to the state shown in FIG. 10F.

As described above, in a case of the needle withdrawal operation, drug injection device 2 drives motor 41 in the reverse direction. When motor 41 rotates, the rotation of motor 41 is transmitted to actuator 5 (specifically, feed screw 50) via decelerator 42 and transmitter 43.

Then, feed screw 50 rotates in the second rotational direction. When feed screw 50 rotates, torque limiter 52 rotates together with feed screw 50.

In this case, since the rotation of slider 51 in the second rotational direction is not restricted, the torque acting on torque limiter 52 is equal to or less than a predetermined value. Therefore, the rotation of feed screw 50 is transmitted to slider 51 via torque limiter 52.

That is, in this state, torque limiter 52 connects feed screw 50 and slider 51.

When slider 51 rotates, guide groove 510 of slider 51 is guided by guide protrusion 310 (see FIG. 9) of casing 3 (specifically, inner case element 31), and slider 51 moves rearward.

Slider 51 moves rearward until guide protrusion 310 is positioned at the start point (front end) of guide groove 510. In this case, slider 51 moves from the front end to the rear end of the stroke of slider 51.

When slider 51 moves rearward, latch 53 and latch cover 54 move rearward together with slider 51. The front end portion of latch 53 is in contact with the rear end portion of syringe 13 (specifically, syringe body 130). Therefore, when latch 53 moves rearward, syringe 13 moves rearward based on the biasing force of spring 12.

When latch cover 54 moves rearward, latch cover 54 moves to a position where device-side engagers 302 in holder 30 of casing 3 is not covered from the outside. As a result, the locking of drug cartridge 1 to drug injection device 2 is released.

In this way, in the present embodiment, when the needle withdrawal operation is performed, the unlocking operation is automatically performed in the course of the needle withdrawal operation.

In addition, feed screw 50 moves rearward in conjunction with the movement of slider 51 rearward. In this case, feed screw 50 moves rearward while rotating.

Then, in a state where slider 51 has moved to the rear end of the stroke of slider 51, guide protrusion 310 of casing 3 (specifically, inner case element 31) is positioned at start point 511 (that is, front end portion) of guide groove 510. In this state, slider 51 is restricted from rotating in the second rotational direction.

At this point, needle 131 of syringe 13 is removed from the user and is accommodated in drug injection device 2. At this point, drug injection device 2 is in the state shown in FIG. 10E. It should be noted that controller 204 may determine that the needle withdrawal operation is completed based on the detection values of first detector 207 and second detector 208 after the needle withdrawal operation of drug injection device 2 is started.

Specifically, controller 204 determines that the needle withdrawal operation is completed when the detection value of first detector 207 is OFF and the detection value of second detector 208 is OFF.

Here, the description will be given again with reference to the flowchart of FIG. 15. In step S402 of FIG. 15, controller 204 determines whether or not the needle withdrawal is completed. The determination method is as described above.

When it is determined that the needle withdrawal is not completed in step S402 of FIG. 15, controller 204 repeats step S402. Then, the needle withdrawal operation is continued.

When it is determined that the needle withdrawal is completed in step S402 of FIG. 15 (the state of FIG. 10E), controller 204 advances the control processing to step S403.

In step S403 of FIG. 15, controller 204 notifies the user of a message. Specifically, controller 204 notifies the user of a message indicating that the needle withdrawal is completed.

In addition, controller 204 notifies the user of a message indicating that drug cartridge 1 is discharged. Such a message may be notified by drug injection device 2 or may be notified by the mobile terminal wirelessly connected to drug injection device 2.

Then, controller 204 continues the needle withdrawal operation of drug injection device 2. Hereinafter, the description will be given again of the needle withdrawal operation of drug injection device 2.

When slider 51 moves to the rear end of the stroke and the rotation of slider 51 in the second rotational direction is restricted, the torque acting on torque limiter 52 from feed screw 50 is larger than the predetermined value.

When the torque acting on torque limiter 52 is larger than the predetermined value, torque limiter 52 is in a state (that is, the non-transmission state of the torque limiter) in which the rotation of feed screw 50 is not transmitted to slider 51. That is, torque limiter 52 disconnects feed screw 50 from slider 51.

In this way, in the course of the needle withdrawal operation of drug injection device 2, slider 51 is restricted from rotating in the second rotational direction. When the rotation of slider 51 is restricted, slider 51 does not move further rearward. As described above, feed screw 50 moves together with slider 51.

Therefore, the rearward movement of feed screw 50 is restricted in the course of the needle withdrawal operation of drug injection device 2. It should be noted that, in a state where the rearward movement of feed screw 50 is restricted, feed screw 50 is positioned at the position shown in FIG. 10E.

In addition, in a state where the rearward movement of feed screw 50 is restricted, feed screw 50 continues to rotate in the second rotational direction based on the power of motor 41. As a result, piston 55 connected to the front end portion of feed screw 50 moves rearward.

Then, piston 55 moves to the basic position shown in FIG. 10F. When piston 55 moves to the basic position, the needle withdrawal operation of drug injection device 2 is ended.

Hereinafter, the description will be given again with reference to the flowchart of FIG. 15. In step S404 of FIG. 15, controller 204 determines whether or not piston 55 has returned to the basic position. Controller 204 determines whether or not piston 55 has returned to the basic position based on the detection value of basic position detector 209. The determination method is as described above.

When it is determined that piston 55 has not returned to the basic position in step S404 of FIG. 15, controller 204 repeats step S404. Then, the needle withdrawal operation of drug injection device 2 is continued.

On the other hand, when it is determined that piston 55 has returned to the basic position in step S404 of FIG. 15, controller 204 advances the control processing to step S405.

In step S405 of FIG. 15, controller 204 stops motor 41. Then, controller 204 ends the needle withdrawal operation of drug injection device 2.

In step S406 of FIG. 15, controller 204 notifies the user of a message. Specifically, controller 204 notifies the user of a message prompting the user to remove the cartridge. Such a message may be notified by drug injection device 2 or may be notified by the mobile terminal wirelessly connected to drug injection device 2.

In step S407 of FIG. 15, the user takes out drug cartridge 1 from drug injection device 2.

In step S408 of FIG. 15, controller 204 determines whether or not drug cartridge 1 is taken out from drug injection device 2. Controller 204 determines whether or not drug cartridge 1 is taken out from drug injection device 2 based on the detection value of cartridge detector 206.

When it is determined that drug cartridge 1 is not taken out from drug injection device 2 in step S408 of FIG. 15, controller 204 repeats the process of step S408.

When it is determined that drug cartridge 1 is taken out from drug injection device 2 in step S408 of FIG. 15, controller 204 ends the control processing.

### (Actions and Effects of Present Embodiment)

According to drug cartridge 1 and drug injection device 2 of the present embodiment having the above-described configuration, a drug cartridge and a drug injection device having excellent portability can be provided.

First, drug cartridge 1 of the present embodiment is mainly composed of four components of case 10, cap 11, spring 12, and syringe 13. Therefore, it is easy to reduce the size of drug cartridge 1. When small drug cartridge 1 is realized, the reduction in size of drug injection device 2 to which drug cartridge 1 is attached can also be realized.

In particular, in the case of drug cartridge 1 of the present embodiment, spring 12 is directly engaged with case 10 and syringe 13 in order to constantly bias syringe 13 rearward. Such a configuration greatly contributes to reducing the number of components.

In addition, in the case of drug injection device 2 of the present embodiment, single motor 41 realizes the above-described locking operation, puncturing operation, injection operation, needle withdrawal operation, and unlocking operation. Such a configuration contributes to a significant reduction in size of drug injection device 2. In addition, such a configuration can simplify the structure of drug injection device 2. As a result, the manufacturing cost of drug injection device 2 can be reduced. In addition, the actions and effects obtained from drug cartridge 1 and drug injection device 2 of the present embodiment are as described above.

The disclosure of Japanese Patent Application No. 2023-146124, filed on September 8, 2023, including the specification, drawings and abstract, is incorporated herein by reference in its entirety. Industrially applicable

The present invention contributes to the realization of a drug cartridge and a drug injection device having excellent portability, and has great industrial applicability.

### Reference Signs List

1 Drug cartridge
10 Case
100a, 100b Flat surface portion
101 RFID tag
102a, 102b Slit
102c Rear end portion
102d Front end portion
103 Locking engager
104 Engagement retainer
105 Spring seat
11 Cap
110 Large-diameter tubular portion
111 Small-diameter tubular portion
112a, 112b Cap-side engager
113 Radial-expansion-allowing space
12 Spring
13 Syringe
130 Syringe body
130a Flange
131 Needle
132 Syringe-side piston
133 Needle shield
2, 2A Drug injection device
200 Position detector
201, 201a Skin detector (first skin contactor)
202 Injection button
203 Main board
204 Controller
205 RFID antenna
206 Cartridge detector
207 First detector
208 Second detector
209 Basic position detector
210 Memory
211 LED light
212 Second skin contactor
3 Casing
30 Holder
301, 301a Cartridge housing
302 Device-side engager
302a Claw portion
31 Inner case element
310 Guide protrusion
4 Driver
40 Power supply
41 Motor
42 Decelerator
43 Transmitter
430 First rotational transmission member
431 Second rotational transmission member
5 Actuator
50 Feed screw
501 Base portion
502 Screw portion
51 Slider
510 Guide groove
511 Start point
512 End point
52 Torque limiter
53 Latch
531 Through-hole
54 Latch cover
541 Detection target
55 Piston
550 Detection target

## Claims

1. A drug cartridge configured to be incorporated into and used with a drug injection device, the drug cartridge comprising:
a syringe configured to accommodate a drug;
a case configured to accommodate the syringe such that the syringe is movable in a front-rear direction;
a cap attached to a front end portion of the case; and
a spring disposed between the syringe and the case and configured to constantly bias the syringe rearward.

2. The drug cartridge according to claim 1, wherein:
the syringe includes a syringe-side engager configured to engage with the spring, and
the case includes:
a first case-side engager configured to engage with the spring, and
a second case-side engager configured to restrict rearward movement of the syringe.

3. The drug cartridge according to claim 2, wherein:
the case includes a third case-side engager configured to restrict forward movement of the syringe, and
the syringe is movable in the front-rear direction by a distance between the second case-side engager and the third case-side engager.

4. The drug cartridge according to claim 3, wherein:
the syringe includes:
a syringe body including a needle at a front end portion, and
a needle shield attached to the front end portion of the syringe body, the cap engages with the needle shield, and
in an operation of removing the cap,
when the cap is pulled forward, the syringe together with the cap moves forward while contracting the spring, and the syringe comes into contact with the third case-side engager, and
when the cap is further pulled forward, the cap is removed from the case, and the needle shield is removed from the syringe body.

5. The drug cartridge according to claim 1, wherein:
the syringe includes:
a syringe body including a needle at a front end portion, and
a needle shield attached to the front end portion of the syringe body, the cap includes a cap-side engager configured to engage with the needle shield, and the case includes:
a radial expansion allowing section configured to allow radial expansion of the cap-side engager when the syringe is inserted into the case, and
a radial expansion preventer configured to prevent radial expansion of the cap-side engager when the cap is removed from the case.

6. A drug injection device, comprising:
a casing including a housing configured to accommodate a drug cartridge configured to hold a syringe;
a motor accommodated in the casing; and
an actuator accommodated in the casing, wherein
the actuator performs, based on power of the motor,
a puncturing operation of moving the syringe in a front-rear direction,
an injection operation of moving a piston of the syringe forward, and
a locking operation of locking the drug cartridge.

7. The drug injection device according to claim 6, wherein
the motor is composed of a single motor.

8. The drug injection device according to claim 6, further comprising:
a cartridge detector configured to detect accommodation of the drug cartridge in the housing, wherein
the actuator performs the locking operation according to a detection result of the cartridge detector.

9. The drug injection device according to claim 6, wherein:
the casing includes a first locking member configured to engage with the drug cartridge, and
the actuator includes a second locking member that is configured to move in the front-rear direction by the motor and prevent, at a first position, release of engagement between the drug cartridge and the first locking member.

10. The drug injection device according to claim 9, wherein:
the first locking member has a tubular shape through which the drug cartridge is insertable, and constitutes the housing,
the second locking member has a tubular shape capable of covering an outer peripheral surface of the first locking member, and
the second locking member covers an engaged portion between the drug cartridge and the first locking member from an outside at the first position.

11. The drug injection device according to claim 6, wherein
the actuator performs the locking operation by rotating the motor by a first predetermined amount in a predetermined direction when the drug cartridge is accommodated in the housing.

12. The drug injection device according to claim 11, wherein
the actuator performs the puncturing operation by rotating the motor in the predetermined direction after the locking operation, and performs the injection operation by rotating the motor in the predetermined direction after the puncturing operation.

13. The drug injection device according to claim 6, wherein
the actuator includes a switching mechanism for switching between the puncturing operation and the injection operation.

14. The drug injection device according to claim 6, wherein:
the actuator includes a first moving member configured to move in the front-rear direction by the motor, and
the syringe moves forward by the first moving member moving forward in the puncturing operation.

15. The drug injection device according to claim 14, wherein:
the first moving member includes a helical guide groove in an outer peripheral surface, and
when the first moving member is rotated by the motor, the first moving member moves forward based on engagement between the helical guide groove and a guide protrusion inserted into the helical guide groove.

16. The drug injection device according to claim 15, wherein
a movement amount of the first moving member in the front-rear direction is a distance between a start point and an end point of the helical guide groove in the front-rear direction.

17. The drug injection device according to claim 16, wherein:
the actuator includes:
a second moving member disposed on a motor side with respect to the first moving member and configured to rotate by the motor in the injection operation to push the drug in the syringe forward, and
a torque limiter disposed between the first moving member and the second moving member, and
the torque limiter connects the first moving member to the second moving member in the puncturing operation and disconnects the first moving member from the second moving member in the injection operation.

18. The drug injection device according to claim 17, wherein
the torque limiter disconnects the first moving member from the second moving member when the first moving member moves to a front end of a stroke.

19. The drug injection device according to claim 17, wherein
after the injection operation, the actuator rotates the motor in reverse to move the syringe rearward, and then performs a needle withdrawal operation in which the second moving member is moved rearward.

20. The drug injection device according to claim 19, wherein
the actuator performs an unlocking operation for the drug cartridge in the course of the needle withdrawal operation.

21. The drug injection device according to claim 6, wherein:
the actuator includes:
a puncturing operation mechanism configured to be actuated by the motor in the puncturing operation,
an injection operation mechanism configured to be actuated by the motor in the injection operation, and
a locking operation mechanism configured to be actuated by the motor in the locking operation.
